# EUROPEAN PATENT SPECIFICATION

(11) **EP 4 545 546 B1**
(45) Date of publication and mention of the grant of the patent: **12.11.2025**
(21) Application number: 23205097.1
(22) Date of filing: 23.10.2023
(51) Int. Cl.: A01N 37/46, A01N 43/52, A01P 7/04

(54) **INSECT NEUROPEPTIDES**
NEUROPEPTIDE FÜR INSEKTEN
NEUROPEPTIDES D'INSECTES

(43) Date of publication of application: 30.04.2025
(60) Divisional application: 25168381.9
(73) Proprietor: Solasta Bio Limited, Glasgow G2 5RZ (GB)
(72) Inventor: DOW, Julian A. T., Glasgow, G61 2JZ (GB); DAVIES, Shireen A., Glasgow, G61 2JZ (GB); ABUL-HAIJA, Yousef, Glasgow, G64 1EB (GB); ARCHIBALD, Lewis, Glasgow, G42 9SD (GB)
(74) Representative: HGF

(56) References cited:
- WO-A1-03/066080
- WO-A2-2020/115076
- KR-B1- 101 842 336
- K�LLISCH GABRIELE V. ET AL: "Structure elucidation and biological activity of an unusual adipokinetic hormone from corpora cardiaca of the butterfly, Vanessa cardui", EUROPEAN JOURNAL OF BIOCHEMISTRY, vol. 267, no. 17, 1 September 2000 (2000-09-01), Hoboken, USA, pages 5502 - 5508, XP093132298, ISSN: 0014-2956, DOI: 10.1046/j.1432-1327.2000.01611.x
- SCHERKENBECK J ET AL: "Insect neuropeptides: Structures, chemical modifications and potential for insect control", BIOORGANIC & MEDICINAL CHEMISTRY, ELSEVIER, AMSTERDAM, NL, vol. 17, no. 12, 15 June 2009 (2009-06-15), pages 4071 - 4084, XP026152222, ISSN: 0968-0896, [retrieved on 20090103], DOI: 10.1016/J.BMC.2008.12.061

## Description

### Field of the Invention

The present invention relates to the uses of natural or natural-like analogues of insect adipokinetic hormone (AKH) neuropeptides having activity against insects, for example hemipteran, dipteran, lepidopteran, blattodean and/or coleopteran insects, such as aphids, moths and fruit flies, as insect control agents (e.g. insecticides) and plant protection agents, and corresponding methods of increasing insect mortality, inhibiting or preventing infestation of a plant by insects, and methods of reducing or treating an insect infestation of a plant, or of reducing insect load on a plant.

### Background

With a global dependence on broad-spectrum insecticides, the damaging effects of which are well documented, there is increasing need for the development of greener, target-specific insecticides with which to protect valuable crop plants. The development and employment of neuropeptides, and their synthetic analogues offers a promising avenue in the drive for greener and target-specific insecticidal agents.

Within insects, neuropeptides are regulatory peptides with functional roles in growth and development, behaviour and reproduction, metabolism and homeostasis, and muscle movement. Insect neuropeptide families are large and include the insect kinins and CAPA (CAPA, CAP2b, CAPA3) neuropeptides, AKH peptides, and the pyrokinin peptides.

Due to their high specificity, insect neuropeptides and their cognate receptors (G-protein coupled receptors, GPCRs) may be developed towards insecticidal agents to selectively reduce the fitness of target pest insects, whilst minimising detrimental environmental impacts, and minimising harm to other non-target species such as pollinators, which have seen dangerously declining population numbers due to pesticide/insecticide use.

There have been attempts in the art to provided modified synthetic analogues of such insect neuropeptides for use as targeted insecticides, which show promising results. However, whilst desirable and effective, these modified peptides may be challenging to bring to market in many territories in which the use of such modified peptides is heavily regulated.

The present invention aims to solve one or more of the above-mentioned problems, and provides novel natural AKH neuropeptides, and natural-like variants or analogues thereof for use as insect control agents and plant protection agents.

KOLLISCH GABRIELE V. ET AL: "Structure elucidation and biological activity of an unusual adipokinetic hormone from corpora cardiaca of the butterfly, Vanessa cardui", EUROPEAN JOURNAL OF BIOCHEMISTRY, vol. 267, no. 17, 1 September 2000, pages 5502-5508, discloses the peptide Vac-AKH, Pyr-LTFTSSWGGK-amide.

WO 03/066080 A1 (BLM GROUP [US]; SCHACTER BERNICE Z [US]; SCHACTER LEE P [US]) 14 August 2003, discloses the undecapeptide ELTFTSSWGGK.

WO 2020/115076 A2 (UNIV GLASGOW COURT [GB]; US AGRICULTURE [US]) 11 June 2020, discloses insect neuropeptides, such as insect kinins and CAPA peptides and discloses a range of peptide analogues as insecticide, insect control agent or as a plant protection agent.

SCHERKENBECK J ET AL: "Insect neuropeptides: Structures, chemical modifications and potential for insect control", BIOORGANIC & MEDICINAL CHEMISTRY, vol. 17, no. 12, 15 June 2009, pages 4071-4084, reviews insect neuropeptides and the potential use for insect control. Table 3 discloses selected myokinins, such as Achetakinin III (ALPFSSWG-amide).

KR 101 842 336 B1 (ANDONG NAT UNIV INDUSTRY-ACADEMIC COOP FOUND [KR]) 28 March 2018, discloses the use of a Se-AKH (adipokinetic hormone) peptide derived from *Spodoptera exigua* having the amino acid sequence AHIYICVYWG as an insect control agent, or as a plant protection agent for protecting a plant or part thereof against insects.

### Summary of the Invention

The inventors have discovered new adipokinetic hormone (AKH) peptides, and natural-like variants or analogues thereof, having insecticidal activity against insects such as hemipteran, dipteran, lepidopteran, blattodean and/or coleopteran insects, and so potentially finding use as pest control agents or insecticides, while having little or no effect against important pollinator species such as bees.

In a first aspect, there is provided the use of a compound having the formula (I) below, or a salt or solvate thereof:

R¹-Y¹-Z-Y²-R² (I)

wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 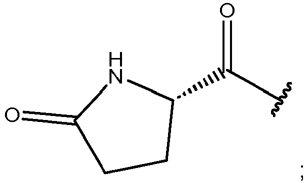 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
   LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) or a composition comprising a compound of formula (I) in admixture with one or more solvents, carriers, diluents, adjuvants, preservatives, dispersants, emulsifying agents, or synergists, as an insect control agent, or as a plant protection agent for protecting a plant or part thereof against insects.

In one embodiment, there is provided the use of a compound or a composition against:
a) dipteran insects, preferably wherein the dipteran insects are selected from: D. *melanogaster or D. suzukii;*
b) hemipteran insects, such as aphids, preferably wherein the hemipteran insects are selected from: *Aphis fabae, Aphis gossypii, Acyrthosiphon pisum, Myzus persicae or Rhopalosiphum padi*; and/or
c) lepidopteran insects, preferably wherein the lepidopteran insects are selected from: *Plutella xylostella* or *Spodoptera frugiperda*;
d) blattodean insects; and/or
e) coleopteran insects,
preferably wherein the use of a compound is against *Plutella xylostella* or *Spodoptera frugiperda* insects.

In one embodiment, there is provided the use of a compound or a composition, wherein R¹ is selected from hydrogen, acyl or fatty acyl, or phosphate or sulphate, or R¹ is a pyroglutamate group of the formula: 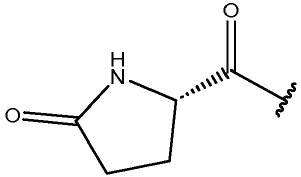 wherein acyl or fatty acyl are optionally substituted by a sugar moiety, phosphate or sulphate.

In one embodiment, there is provided the use of a compound or a composition, wherein R¹ is selected from hydrogen, formyl, acetyl (Ac), propanoyl, butanoyl, palmitoyl, butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, valeryl, and a pyroglutamate group of the formula: 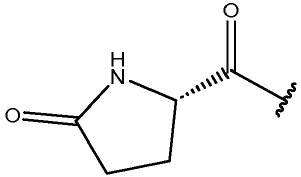
preferably wherein R¹ is selected from hydrogen, or palmitoyl, or pyroglutamate, more
preferably wherein R¹ is pyroglutamate.

In one embodiment, there is provided the use of a compound or a composition, wherein:
Y¹ is absent; and/or
wherein Y² is present and is selected from: GR, RG, KR, RK, K, R, and G; and/or
wherein R² is NH₂.

In one embodiment, there is provided the use of a compound or a composition, wherein the compound has the formula:
[Pyr]-LTFTSSWGG-[NH₂] (SEQ ID NO:2).

In a second aspect, there is provided a method of increasing insect mortality, the method comprising contacting an insect or insect population with a compound having the formula (I) below, or a salt or solvate thereof:

R¹-Y¹-Z-Y²-R² (I)

wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 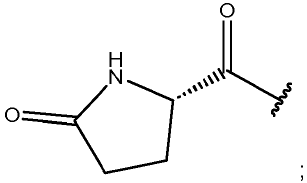 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
   LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

In third aspect, there is provided a method of inhibiting or preventing infestation of a plant by insects, the method comprising contacting the plant or a part thereof, or a site on which the plant is growing or is intended to be grown, with a compound having the formula (I) below, or a salt or solvate thereof:

R¹-Y¹-Z-Y²-R² (I)

wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 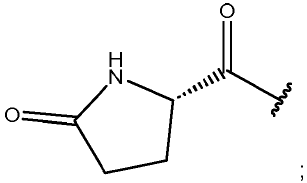 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
   LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

In one embodiment, there is provided the method, wherein the compound or composition is contacted with the plant or part thereof, or a site on which the plant is growing or is intended to be grown, while the plant or part is free or substantially free of insects.

In a fourth aspect, there is provided a method of reducing or treating an insect infestation of a plant, or of reducing insect load on a plant, the method comprising contacting the plant or a part thereof, or a site on which the plant is growing, with a compound having the formula (I) below, or a salt or solvate thereof:

R¹-Y¹-Z-Y²-R² (I)

wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 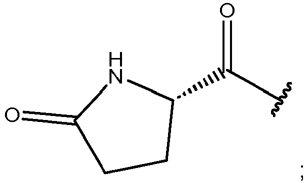 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
   LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

In one embodiment of the second, third and fourth aspects, R¹ is selected from hydrogen, acyl or fatty acyl, or phosphate or sulphate, or R¹ is a pyroglutamate group of the formula: 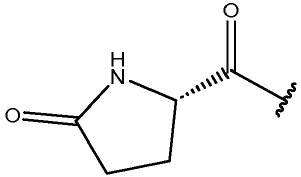 wherein acyl or fatty acyl are optionally substituted by a sugar moiety, phosphate or sulphate.

In one embodiment of the second, third and fourth aspects, R¹ is selected from hydrogen, formyl, acetyl (Ac), propanoyl, butanoyl, palmitoyl, butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, valeryl, and a pyroglutamate group of the formula: 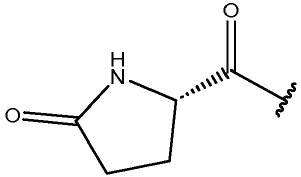
preferably wherein R¹ is selected from hydrogen, or palmitoyl, or pyroglutamate, more
preferably wherein R¹ is pyroglutamate.

In one embodiment of the second, third and fourth aspects:
Y¹ is absent; and/or
wherein Y² is present and is selected from: GR, RG, KR, RK, K, R, and G; and/or
wherein R² is NH₂.

In one embodiment of the second, third and fourth aspects, the compound has the formula:
[Pyr]-LTFTSSWGG-[NH₂] (SEQ ID NO:2).

In one embodiment of the third aspect, the site on which the plant is growing or intended to be grown may comprise an agricultural site suitable for growing plants, preferably the site comprises a field.

In one embodiment of the second, third and fourth aspects, the insects are:
a) dipteran insects, preferably wherein the dipteran insects are selected from: D. *melanogaster or D. suzukii;*
b) hemipteran insects, such as aphids, preferably wherein the hemipteran insects are selected from *Aphis fabae, Aphis gossypii, Acyrthsiphon pisum, Myzus persicae* or *Rhopalosiphum padi;*
c) lepidopteran insects, preferably wherein the lepidopteran insects are selected from: *Plutella xylostella* or *Spodoptera frugiperda;*
d) blattodean insects; and/or
e) coleopteran insects,
preferably wherein the insects are *Plutella xylostella* or *Spodoptera frugiperda* insects.

In one embodiment of the second, third and fourth aspects, contacting the insect or insect population, plant or part thereof, or site, comprises watering feeding, spraying, atomizing, foaming, fogging, culturing in hydroculture, culturing in hydroponics, coating, submerging, injecting and/or encrusting the insect or insect population, plant or part thereof, or site with the compound or composition.

In one embodiment of the second, third and fourth aspects, the insect or insect population, plant or part thereof, or site, is contacted with an effective concentration of the compound, preferably a concentration of between 10⁻³ to 10⁻⁹ M.

In one embodiment of any aspect, the composition is an aqueous composition.

In one embodiment of any aspect, the composition comprises an effective amount of the compound.

Subject matter referred to herein as being "further described herein", "of the disclosure", "in some/certain examples" is not necessarily part of the claimed invention as such, which is defined by the claims, but is considered as useful for understanding or interpreting the invention.

Further described herein is an insecticidal compound having the formula (I) below, or a salt or solvate thereof:

R¹-Y¹-Z-Y²-R² (I)

wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 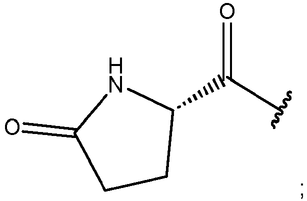 and any alkyl, formyl, acyl or fatty acyl may optionally be substituted with one or more groups selected from oxo or C₁₋₆alkyl or a sugar moiety, phosphate or sulphate;
Y¹ is absent or is a peptide comprising from 1 to 2 amino acids;
Z is a peptide according to formula
   LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl).

Further described herein is a composition comprising the compound as defined herein, or a salt thereof, in admixture with one or more solvents, carriers, diluents, adjuvants, preservatives, dispersants, emulsifying agents, or synergists. In some examples the composition may be an agricultural composition, an insect control composition or plant protection composition.

Further described herein is a method of increasing hemipteran, dipteran, lepidopteran, blattodean and/or coleopteran insect mortality, the method comprising contacting a hemipteran, dipteran, lepidopteran, blattodean and/or coleopteran insect population with a compound as defined herein, or a salt thereof, or a composition as defined herein. In some examples the insect may be a lepidopteran insect such as moths.

Further described herein is a method of inhibiting infestation of a plant by hemipteran, dipteran, coleopteran, blattodean and/or lepidopteran insects comprising contacting the plant with a compound as defined herein, or a salt thereof, or a composition as defined herein. Suitably, the compound or composition is applied to the plant while the plant is free or substantially free of hemipteran, dipteran, lepidopteran, blattodean and/or coleopteran insects. In some examples the insects are selected from hemipteran, dipteran and/or lepidopteran insects, in some examples lepidopteran insects such as moths.

Further described herein is a method of reducing hemipteran, dipteran, lepidopteran, blattodean and/or coleopteran insect infestation of a plant, or of reducing hemipteran, dipteran, lepidopteran, blattodean and/or coleopteran insect load on a plant, the method comprising contacting the plant with a compound as defined herein, or a salt thereof, or a composition as defined herein. In some examples the insects may be selected from hemipteran, dipteran and/or lepidopteran insects, most preferably lepidopteran insects such as moths. In some examples the compounds, compositions, and agents of the disclosure suitably have activity against the moth species *Plutella xylostella.* In some examples the compounds, compositions, and agents of the disclosure suitably have activity against the moth species *Spodoptera frugiperda.*

In some examples insects which encode the AKH peptide include hemipteran, dipteran, lepidopteran, blattodean and/or coleopteran insects, preferably hemipteran, dipteran and/or lepidopteran insects. In some examples the insects which encode the AKH peptide may be lepidopteran insects such as moths.

Further described herein is a method of producing an insecticidal compound disclosed herein, the method comprising:
(a) chemically synthesising the insecticidal compound of formula (I) or a precursor thereof.

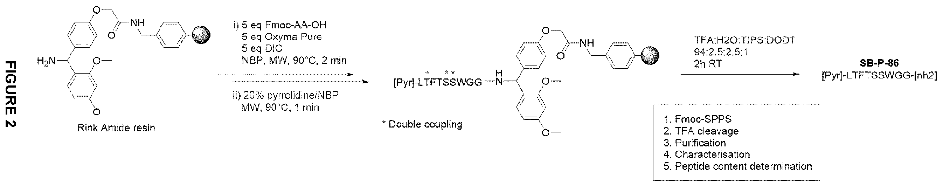

Such chemical synthesis may comprise the method described in the examples. Such chemical synthesis may comprise the method and steps shown in Figure 2.

Further features will now be defined under the following headed sections.

### DETAILED DESCRIPTION

### Definitions

Throughout the present description and claims the conventional three-letter and one-letter codes for naturally occurring amino acids are used, i.e. A (Ala), G (Gly), L (Leu), I (lle), V (Val), F (Phe), W (Trp), S (Ser), T (Thr), Y (Tyr), N (Asn), Q (Gln), D (Asp), E (Glu), K (Lys), R (Arg), H (His), M (Met), C (Cys) and P (Pro).

'Amino acid' as referred to herein may refer to a naturally occurring amino acid or any other amino acid including synthetic amino acids, and non-proteinogenic amino acids. By "naturally occurring" in this context is meant the 20 amino acids encoded by the standard genetic code, sometimes referred to as proteinogenic amino acids.

The term amino acid is short for α-amino [alpha-amino] carboxylic acid. Each molecule contains a central carbon atom, called the α-carbon, to which both an amino and a carboxyl group are attached. The remaining two bonds of the α-carbon atom are generally satisfied by a hydrogen (H) atom and the side chain, shown as R below:

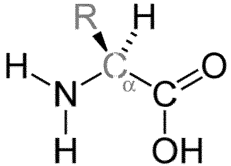

Unless otherwise specified, amino acid residues in peptides of the disclosure are of the L-configuration. As used herein, the terms "polypeptide", "protein", "peptide", are used interchangeably, and refer to a polymeric form of amino acids of any length, which can include coded and non-coded amino acids. As used herein, amino acid residues will be indicated as above either by their full name or according to the standard three-letter or one-letter amino acid code.

The notation Cₓ₋ₓₓ refers to the number of carbon atoms in a functional group. The number in the 'x' positions is the lowest number of carbon atoms and the number in the 'xx' position denotes the highest number of carbon atoms. For example, C₁₋₆-alkyl refers to alkyl groups as defined herein having from 1 to 6 carbon atoms.

The notation *l, n* or *t* are used herein in relation to various alkyl groups in the normal way. Specifically, the suffixes refer to the arrangement of atoms and denote straight chain ('*n*') or branched (*'i'* or *'t'*) alkyl groups.

The term "alkyl" as used herein refers to a saturated linear or branched-chain monovalent hydrocarbon radical, wherein the alkyl radical may be optionally substituted. The number of carbon atoms in the alkyl group may be specified using the above notation, for example, when there are from 1 to 8 carbon atoms the term "C₁₋₈-alkyl" may be used. Examples of alkyl groups include methyl (Me, -CH₃), ethyl (Et, -CH₂CH₃), 1-propyl (*n*-Pr, *n*-propyl, -CH₂CH₂CH₃), 2-propyl (*i*-Pr, *i*-propyl, -CH(CH₃)₂), and 1-butyl (*n*-Bu, *n*-butyl, - CH₂CH₂CH₂CH₃).

An "alkylene," "alkenylene," or "alkynylene" group is an alkyl, alkenyl, or alkynyl group that is positioned between and serves to connect two other chemical groups. Thus, "C₁₋₆alkylene" means a linear saturated divalent hydrocarbon radical of one to six carbon atoms or a branched saturated divalent hydrocarbon radical of three to six carbon atoms. Example alkylene groups include methylene (-CH₂-), 1,1-ethylene (-CH(CH₃)-), 1,2-ethylene (-CH₂CH₂-), 1,1-propylene (-CH(CH₂CH₃)-), and 2,2-propylene (-C(CH₃)₂-).

The term "alkenyl" as used herein refers to a linear or branched-chain monovalent hydrocarbon radical with at least one site of unsaturation, i.e., a carbon-carbon double bond. The alkenyl radical may be optionally substituted, and includes radicals having "cis" and "trans" orientations, or alternatively, "E" and "Z" orientations. The number of carbon atoms in the alkenyl group may be specified using the above notation, for example, when there are from 2 to 8 carbon atoms the term "C₂₋₈-alkenyl" may be used. Example alkenyl groups include, but are not limited to, ethenyl (-CH=CH₂), and prop-1-enyl (-CH=CHCH₃).

In the chemical structures drawn herein, the presence of " 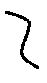 " or " 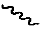 " denotes a point of attachment or a radical for example, a radical as discussed in relation to various functional groups.

The term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "about" as used herein when referring to a measurable value such as a parameter, an amount, a temporal duration, and the like, is meant to encompass variations of +/-10% or less, preferably +/-5% or less, more preferably +/- 1% or less, and still more preferably +/- 0.1% or less of and from the specified value, insofar such variations are appropriate to perform. It is to be understood that the value to which the modifier 'about' refers is itself also specifically, and preferably, disclosed.

"Plant" as used herein, means an entire plant or a part thereof, including fresh fruit, vegetables and seeds. The plant or plant part may be a live plant or part thereof. Also, the term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

"Crop" as used herein means a plant species or variety that is grown to be harvested as food, livestock fodder, fuel raw material, or for any other economic purpose. As a non-limiting example, said crops can be maize, cereals, such as wheat, rye, barley and oats, sorghum, rice, sugar beet and fodder beet, fruit, such as pome fruit (e.g. apples and pears), citrus fruit (e.g. oranges, lemons, limes, grapefruit, or mandarins), stone fruit (e. g. peaches, nectarines or plums), nuts (e.g. almonds or walnuts), soft fruit (e.g. cherries, strawberries, blackberries or raspberries), the plantain family or grapevines, leguminous crops, such as beans, lentils, peas and soya, oil crops, such as sunflower, safflower, rapeseed, canola, castor or olives, cucurbits, such as cucumbers, melons or pumpkins, fibre plants, such as cotton, flax or hemp, fuel crops, such as sugarcane, miscanthus or switchgrass, vegetables, such as potatoes, tomatoes, peppers, lettuce, spinach, onions, carrots, egg-plants, asparagus or cabbage, ornamentals, such as flowers (e.g. petunias, pelargoniums, roses, tulips, lilies, or chrysanthemums), shrubs, broadleaved trees (e.g. poplars or willows) and evergreens (e.g. conifers), grasses, such as lawn, turf or forage grass or other useful plants, such as coffee, tea, tobacco, hops, pepper, rubber or latex plants.

A "pest", as used here, is an organism that is harmful to plants, animals, humans or human concerns, and includes, but is not limited to crop pests such as insects (as later defined), household pests or insects, such as cockroaches, ants, etc., and disease vectors, such as malaria mosquitoes.

"Pest infection" or "pest disease" as used herein refers to any inflammatory condition, disease or disorder in a living organism, such as a plant, animal or human, which is caused by a pest.

"Active substance", "active ingredient" or "active principle", as used interchangeably herein, means any biological, biochemical or chemical element and its derivatives, fragments or compounds based thereon, including micro-organisms, having general or specific action against harmful organisms on a subject, and in particular on insect pests of plants, parts of plants or on plant products, as they occur naturally or by manufacture, including any impurity inevitably resulting from the manufacturing process.

The terms "effective amount" and "effective dose", as used herein, mean the amount needed to achieve the desired result or results.

An "insect", as used here, is used in the broad popular sense and includes all species of the superphylum Panarthropoda (classification Systema Naturae, Brands, S.J. (comp.) 1989- 2005. Systema Naturae 2000. Amsterdam, The Netherlands, [http://sn2000.taxonomy.nl/]), including the phyla Arthropoda, Tardigrada and Onychophora; it includes all the different phases of the life cycle, such as, but not limited to eggs, larvae, nymphs, pupae and adults. Suitable insect pests are defined elsewhere herein.

An "insecticidal compound" as used herein refers to compounds having biological activity on insects (as defined above), including but not limited to compounds capable of killing the insect, larvaecides, insect growth regulators, behaviour modifying compounds, attractants, repellents, pheromones, kairomones, allomones and entomopathogenic fungi, viruses and proteins. The insecticide exerts its biological activity preferably by the contact of the compound with the insect, without the need of being ingested by the insect. It includes not only compounds or compound formulations that are ready to use, but also precursors in an inactive form, which may be activated by outside factors. Possibly, the insecticide may be combined with materials used in conjunction, such as synergists or safeners, flavour or odour compositions. Preferably, said compound is comprised in a carrier as defined above. "Comprised in a carrier" as used herein means bound on or contained in by means such as but not limited to embedding, encapsulation and adsorption.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another example includes from the one particular value and/or to the other particular value. Similarly, when values are expressed as approximations, by the use of the antecedent "about," it will be understood that the particular value forms another example. The term "about" in relation to a numerical value is optional and means for example +/- 10%.

### Compounds

Particular compounds of the disclosure include, for example, compounds of the formula (I), wherein, unless otherwise stated, each of R¹, Y¹, Y², R² and any associated substituent group has any of the meanings defined hereinbefore or in any of paragraphs (1) to (12) hereinafter:-
(1) R¹ is selected from hydrogen, acyl or fatty acyl, or phosphate or sulphate, or R¹ is a pyroglutamate group of the formula: 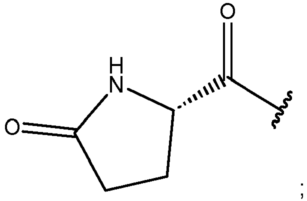 wherein acyl or fatty acyl are optionally substituted by a sugar moiety, phosphate or sulphate.
(2) R¹ is selected from hydrogen, formyl, acetyl (Ac), propanoyl, butanoyl, palmitoyl [palm], butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, and valeryl.
(3) R¹ is selected from hydrogen, acetyl or palmitoyl, or R¹ is a pyroglutamate group of the formula: 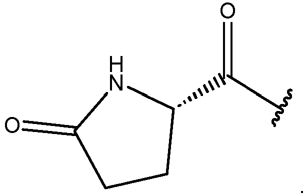
(4) R¹ is selected from hydrogen or palmitoyl.
(5) R¹ is hydrogen.
(6) R¹ is a pyroglutamate group of the formula: 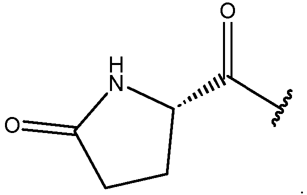
(7) Y¹ is absent or a peptide comprising 1 or 2 amino acid residues.
(8) Y¹ is absent.
(9) Y² is absent or a peptide comprising 1 or 2 amino acid residues.
(10) Y² is absent.
(11) R² is NH₂, NR^{2a}H, NR^{2a}R^{2b} or OH wherein each of R^{2a} and R^{2b} if present is independently C₁₋₆-alkyl (e.g. methyl, ethyl, propyl, butyl, pentyl or hexyl).
(12) R² is NH₂.

Suitably, R¹ may be as defined in any one of paragraphs (1) to (6) above. More suitably, R¹ may be as defined in any one of paragraphs (3) to (6) above. Most suitably, R¹ may be as defined in paragraph (4) or (5) or (6) above.

Suitably, Y¹ or Y² may be as defined in any one of paragraphs (7) to (10) above. Most suitably, Y¹ or Y² may be as defined in paragraphs (8) or (10) above.

Suitably, R² may be as defined in paragraph (11) or (12) above. Most suitably, R² may be as defined in paragraph (12) above.

In one example, R¹ is pyroglutamate, Y¹ and Y² are absent, and R² is NH₂.

In one example, R¹ is pyroglutamate, Y² is absent, Y¹ is one or two amino acids, and R² is NH₂.

In one example, R¹ is pyroglutamate, Y¹ is absent, Y² is one or two amino acids, and R² is NH₂.

### Further Explanation of Compounds

### R¹ and R²

The terminal groups present at the N- and C-termini of the peptide backbone are designated R¹ and R² respectively. Thus, R¹ is bonded to the nitrogen atom of the N-terminal amino group of Y¹ and R² is bonded to the C-terminal carbonyl carbon atom of Y².

The compounds of the disclosure may include further functionalisation, suitably at the N or C terminus. Suitably, the compounds may be functionalised to increase cuticle permeability or to increase stability. Suitably, the compound may be functionalised with an aromatic, aliphatic or lipophilic group. Suitably therefore, R¹ may be an aromatic, heteroaromatic, aliphatic or lipophilic group.

In certain examples, the compound may be functionalised with a lipophilic group such as a fatty acyl group. Fatty acyl groups include but are not limited to palmitoyl, butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, and valeryl. Suitably therefore the R¹ group may be palmitoyl ([Palm]), i.e.:

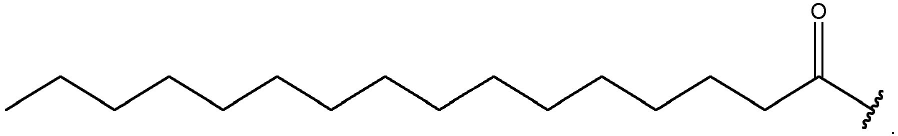

In one example, the compound may be functionalised with an acyl group. An "acyl" group is a group of the formula R^{3a} -C(O)- group wherein R^{3a} is a C₁₋₆alkyl, for example formyl, acetyl (Ac), propanoyl, butanoyl, or wherein R^{3a} is benzoyl. Suitably, the R^{3a} group may be R^{1b}-C(O)-, such as acetyl (Ac), i.e.:

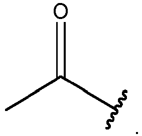

In certain examples, the R¹ group may be substituted with a sugar moiety.

In certain examples, one or more amino acid residues in peptides Y and Z may be naturally modified with a sugar moiety, i.e. the amino acid residue may be a "sugar modified analogue". For example, a sugar may be part of a Ser or Thr side chain modification (glycosylation) or a Lys or Arg side chain modification (glycation).

The sugar moieties discussed herein may be a monosaccharide or disaccharide. Examples of monosaccharides include glucose, 6- deoxyglucose, mannose, galactose, glucosamine, galactosamine, N- acetylglucosamine, N-acetylgalactosamine, glucuronic acid, allose, altrose, gulose, idose, fucose, talose, ribose, deoxyribose, arabinose, xylose, lyxose, ribulose, xylulose, fructose, psicose, sorbose or tagatose. Examples of disaccharides include sucrose, lactose, lactulose, allolactose, maltose, isomaltose, isomaltulose, trehalose, cellobiose, kojibiose, nigerose, sophorose, laminaribiose, gentiobiose, thiomaltose, mannobiose or their N-, C- or S-interglycosidic derivative. Most suitably, the sugar moiety is selected from glucosamine or galactosamine. The sugar can be present as an N-terminus modification or as part of Ser sidechain modification. In certain examples, the R¹ group may be substituted with a phosphate or a sulphate.

In certain examples, one or more amino acid residues in peptides Y and Z may be naturally modified with a phosphate or sulphate. For example the phosphate or sulphate may be part of a side chain modification i.e. the amino acid may be phosphorylated or sulphated.

R¹ may be selected from hydrogen (which may not be noted in the specific peptide sequence, or may instead be designated "H-" or "Hy-"), C₁₋₄ alkyl (e.g. methyl, ethyl, propyl, butyl), -N(R^{1a})-C(=N⁺(R^{1b})(R^{1c}))NR^{1d}R^{1e}, or -C(=N⁺(R^{1b})(R^{1c}))NR^{1d}R^{1e}; wherein each of R^{1a}, R^{1b}, R^{1c}, R^{1d} and R^{1e} is independently selected from hydrogen or C₁₋₄ alkyl (e.g. methyl, ethyl, propyl, butyl), preferably hydrogen or methyl.

In some examples, if R¹ is -C(=N⁺(R^{1b})(R^{1c}))NR^{1d}R^{1e}; each of R^{1a}, R^{1b}, R^{1c}, R^{1d} and R^{1e} are methyl, i.e. R¹ is -C(=N⁺Me₂)NMe₂.

When R¹ = "H" (or "Hy";), it typically indicates a free primary amino group at the N-terminus. The other hydrogen atom of the N-terminal amino group is typically invariant, regardless of the nature of R¹. Exceptionally, when the residue at the N-terminus is N-methylated, R¹ may still be indicated as H even though the N-terminal residue has a secondary amine group. Thus an N-methylated leucine residue at the N-terminus may be indicated as R¹-[n-me-L]- where R¹ is H. However, it could also be shown as simply R¹-L- where R¹ is methyl and the other hydrogen atom is not shown.

In some examples, an N-terminal glutamine (Gln or Q) or N-terminal glutamic acid (Glu or E) residue may undergo a conversion to form the pyroglutamate terminal group, [pyr]: 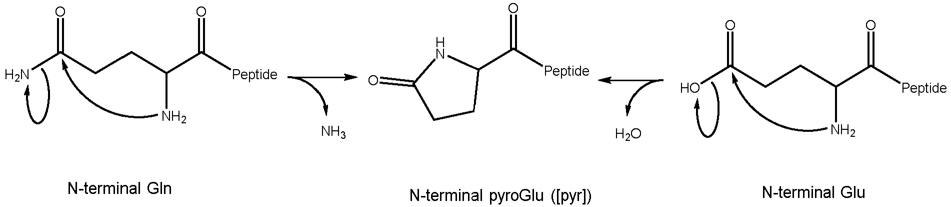 An N-terminal glutamine (Q) or glutamic acid (E) residue may undergo such a conversion in biological systems via reaction with certain enzymes. The conversion may also be achieved synthetically.

The Y¹ peptide, if present, or the Z peptide may therefore also include an N-terminal [pyr] group: 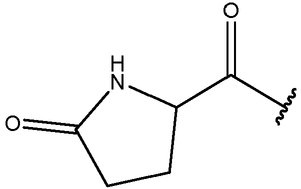 An N-terminal [pyr] group may be formed as a result of cyclisation of an N-terminal glutamine (Gln or Q) or N-terminal glutamic acid (Glu or E) residue to form the pyroglutamate terminal group. Since this conversion may occur under certain biological conditions, it is considered to be a naturally occurring modification of an N-terminal glutamine (Gln or Q) or N-terminal glutamic acid (Glu or E) residue.

Suitably, R² may be NH₂, NR^{2a}H, NR^{2a}R^{2b}, or OR^{2a}; wherein each of R^{2a} and R^{2b} are as defined herein. Preferably R² is NH₂.

A particular example of an AKH peptide according to the disclosure includes:

| | |
|---|---|
| SB-P-86 | [Pyr]-LTFTSSWGG-[NH₂] (SEQ ID NO:2) |

or a salt or solvate thereof.

In some examples, the compounds of the disclosure may be in the form of a salt or solvate (e.g. a hydrate).

The compounds of the disclosure may be provided in combination with one or more additional active insecticides, such as those described herein.

### Y¹ and Y²

The peptide may comprise one or two further peptide groups located between the terminal R¹ and R² groups at the N and C terminus respectively. Suitably these groups are the Y¹ and Y² group. If present, these groups are located on either side of the Z peptide group.

In one example, the Y¹ and Y² groups are absent.

In one example, the compound comprises a Y¹ group only. In one example, the compound comprises a Y² group only.

In one example, the compound comprises a Y¹ and Y² group.

Suitably the Y groups when present comprise one or two amino acids. Suitably the Y groups may comprise any amino acid, suitably selected from: M, R, G, and K.

In one example, the Y groups are selected from: GR, RG, KR, RK, K, R, M, and G. In one example, the Y groups are selected from: RG, KR, and K.

In one example, the compound comprises a Y² group only, wherein the Y² group is selected from RG, KR, and K.

### Modified amino acid or non-natural amino acid analogue

In some examples, the insecticidal compound does not comprise any modified amino acid or non-natural amino acid analogues.

In particular, preferably peptide Y¹, if present does not comprise any modified amino acid or non-natural amino acid analogues, and peptide Y² if present does not comprise any modified amino acid or non-natural amino acid analogues. Thus, in certain examples, the peptides Y¹ and Y² comprise only unmodified amino acid residues, suitably only natural amino acid residues.

In particular, peptide Z does not comprise any modified amino acid or non-natural amino acid analogues. Thus, in certain examples, the peptide Z comprises only unmodified amino acid residues, suitably only natural amino acid residues.

### Activity

Suitably, the compounds of the disclosure have activity against insects. Suitably the activity is against hemipteran insects, dipteran insects, lepidopteran insects, blattodean insects and/or coleopteran insects.

In one example, the compounds of the disclosure have activity against lepidopteran insects. The compounds of the disclosure may therefore find particular use against lepidopteran insects, and find particular application in the associated uses and methods described herein. Particularly preferred lepidopteran insects are moths.

The compounds of the disclosure typically increase insect mortality, for example when contacted topically to a suitable insect, or ingested by a suitable insect. Thus, the compounds of the disclosure described herein (and compositions containing them) may be regarded as insecticides, and may be referred to as "insect control agents".

Suitably the compounds of the disclosure thereby indirectly increase plant health, including increasing plant growth, yields, and the like, due to preventing insect infestations of plants and reducing insect load on plants. Thus, the compounds of the disclosure described herein (and compositions containing them) may also be regarded as "plant protection agents".

Without wishing to be bound by theory, any or all of the effects described may be mediated by agonist activity at the AKH receptor of the target insects. The AKH receptors believed to be present in target insects include the rhodopsin-like GPCR AKH receptor, AKHR. Suitably the compounds of the disclosure bind to one or more AKH receptors of target insects. Suitably the compounds of the disclosure have agonist activity when bound to an AKH receptor of a target insect. Suitably the compounds of the disclosure bind to AKH receptor (AKHR). Suitably the compounds of the disclosure are agonists of AKH receptor (AKHR).

### Insect control agent

The term "insect control agent" refers to agents used to increase insect mortality (i.e. as insecticides). Any compound or composition thereof of the disclosure may be considered as an insect control agent, and may be used as an insect control agent. Suitably therefore further described herein is the use of a compound or composition of the disclosure as an insect control agent, for example against diptera insects, hemiptera insects, coleoptera insects, blattodea insects and/or lepidoptera insects. Thus an insect control agent may be administered to increase mortality of a given insect or insect population.

An increase in mortality used herein is intended to refer to an increase in the percentage of dead insects, as compared to the percentage of dead insects of an otherwise identical insect population which have not been exposed to the insect control agent of the disclosure.

Suitably, insect mortality may be calculated as number of dead insects/total number of insects per treated area. Suitably the treated area may be a well of a plate, or may be one or more leaves, or an entire plant. Suitably insect mortality may be measured by performing a leaf dip assay or lepidoptera artificial diet assay as described herein in the examples.

An insect control agent may be used to reduce the size of an insect population, or inhibit growth of an insect population or inhibit feeding of an insect population (e.g. as compared to an otherwise identical insect population not exposed to the agent).

An insect control composition is a composition comprising an insect control agent i.e. a compound of the disclosure as described.

### Plant protection agent

The term "plant protection agent" refers to agents used to protect a plant or plant part against insects, e.g. against infestation or colonisation, or being used as a food source by such insects (e.g. by the draining of sap). Any compound or composition thereof of the disclosure may be considered as a plant protection agent, and may be used as a plant protection agent. Suitably therefore further described herein is the use of a compound or composition of the disclosure as a plant protection agent, for example to protect a plant against diptera insects, hemiptera insects, coleoptera insects, blattodea insects and/or lepidoptera insects.

Infestation or colonisation may be by larvae (or nymphs), by adult insects, or may be by being used as a host or repository for eggs. The terms "infestation" and "colonisation" should not be construed as requiring the presence of the insects to be deleterious to the plant, however.

A plant protection agent may be applied *inter alia* for reducing insect load on a plant or plant part, for inhibiting or reducing infestation of a plant by insects, for inhibiting (e.g. reducing the rate of) an increase in insect load on a plant or plant part, or for maintaining a plant in an insect-free state, as compared to an otherwise identical plant having an insect population not exposed to the agent. Thus, the plant protection agent may be applied to a plant or plant part which already carries hemipteran, dipteran, coleopteran, blattodean and/or lepidopteran insects, or to a plant or plant part which is free or substantially free of hemipteran, dipteran, coleopteran, blattodean and/or lepidopteran insects.

A plant protection composition is a composition comprising a plant protection agent i.e. a compound of the disclosure as described.

### Plants

By 'plant or plant part', or 'plant or part thereof' referred to herein it is meant any part of a plant including but not limited to; the leaf, stem, root, flower, bud, bulb, and seed.

Suitable plants or parts thereof which may be protected by the compounds or compositions thereof of the disclosure, or by the agents of the present disclosure include crops and plants of agricultural, horticultural, or economic significance. Suitable plants may include any of the following or parts thereof: *Musa textilis, Medicago sativa, Prunus dulcis, Pimpinella anisum, Malus sylvestris, Prunus armeniaca, Areca catechu, Arracacia xanthorhiza, Maranta arundinacea, Cynara scolymus, Helianthus tuberosus, Asparagus officinalis, Persea americona, Pennisetum americanum, Vigna subterranean, Musa paradisiaca, Hordeum vulgare, Phaseolus vulgaris, Phaseolus vigna spp., Beta vulgaris, Citrus bergamia, Rubus spp., Piper nigrum, Acacia mearnsii, Vaccinium spp., Bertholletia excelsa, Artocarpus altilis, Vicia faba, Brassica oleracea botrytis, Sorghum bicolor, Brassica oleracea gemmifera, Fagopyrum esculentum, Brassica oleracea capitate, Brassica rapa, Brassica spp., Theobroma* cacao, *Cucumis melo, Carum carvi, Elettaria cardamomum, Cynara cardunculus, Ceratonia siliqua, Daucus carota, Anacardium occidentale, Manihot esculenta, Ricinus communis, Brassica oleracea botrytis, Apium graveolens, Sechium edule, Prunus spp., Castanea sativa, Cicer arietinum, Cichorium intybus, Cichorium intybus, Capsicum spp., Cinnamomum verum, Cymbopogon nardus, Citrus medica, Citrus veticulata, Trifolium spp., Syzygium aromaticum, Cocos nucifera, Colocasia spp.; Xanthosoma spp., Coffee spp., Cola spp., Brassica napus, Zea mays, Valerianella locusta, Gossypium spp., Vigna unguiculate, Vaccinium spp., Lepidium sativum, Cucumis sativus, Ribes spp., Annona reticulata, Colocasia esculenta, Phoenix dactylifera, Moringa oleifera, Phaseolus spp., Allium sativum, Allium cepa, Pisum sativum, Triticum durum, Xanthosoma spp.; Colocasia spp., Solanum melongena, Cichorium endivia, Lygeum spartum, Foeniculum vulgare, Trigonella foenumgraecum, Ficus carica, Corylus avellane, Furcraea macrophylla, Linum usitatissimum, Phormium tenax, Pelargonium spp.; Geranium spp., Zingiber officinalis, Langenaria spp; Cucurbita spp., Cicer arietinum, Citrus paradise, Vitis vinifera, Lygeum spartum, Dactylis glomerata, Arachis hypogaea, Psidium guajava, Corylus avellane, Cannabis sativa, Crotalaria juncea, Agave fourcroydes, Lawsonia inermis, Humulus lupulus, Armoracia Rusticana, Indigofera tinctorial, Jasminum spp., Corchorus spp., Brassica oleracea acephala, Ceiba pentandra, Hibiscus cannabinus, Brassica oleracea gongylodes, Lavandula spp., Allium ampeloprasum, Citrus limon, Cymbopogon citratus, Lens culinaris, Lespendeza spp., Lactuca sativa, Glycyrrhiza glabra, Citrus aurantifolia, Citrus limetta, Linum usitatissimum, Litchi chinensis, Eriobotrya japonica, Lupinus spp., Macadamia spp., Myristica fragrans, Agave atrovirens, Citrus reticulata, Mangifera indica, Manihot esculenta, Secale cereal, Mespilus germanica, Cucumis melo, Penicum miliaceum, Eleusine coracana, Setaria italica, Echinochloa crusgalli, Eleusine coracana; Mentha spp., Morus spp., Morus alba, Agaricus spp.; Pleurotus spp. Volvariella, Brassica nigra; Sinapis alba, Prunus persica, Phormium tenax, Guizotia abyssinica, Myristica fragrans, Avena spp., Elaeis guineensis, Abelmoschus esculentus, Olea europea, Papaver somniferum, Citrus sinensis, Citrus aurantium, Dactylis glomerate, Metroxylon spp., Borassus flabellifer, Carica papaya, Pastinaca sativa, Pyrus communis, Pisum sativum, Carya illinoensis, Capsicum annuum, Diospyros kaki; Diospyros virginiana, Cajanus cajan, Ananas comosus, Pistacia spp., Prunus domestica, Punica granatum, Citrus grandis, Solamum tuberosum, Ipomoea batatas, Cucurbita spp., Chrysanthemum cineraraiefolium, Aspidosperma spp., Cydonia oblonga, Cinchona spp., Chenopodium quinoa, Raphanus sativus (including Cochlearia armoracia), Boehmeria nivea, Agrostis spp., Boehmeria nivea, Rheum spp., Oryza sativa; Oryza glaberrima, Rose spp., Hevea brasiliensis, Secale cereal, Lolium spp., Carthamus tinctorius, Metroxylon spp., Onobrychis viciifolia, Valerianella locusta, Tragopogon porrifolius, Achras sapota, Citrus reticulata, Brassica ileracea capitate, Scorzonera hispanica, Sesamum indicum, Butyrospermum paradoxum, Agave sislana, Citrus aurantifolia, Glycine max, Triticum spelta, Spinacia oleracea, Secale cereal, Cucurbita spp., Fragaria spp., Sorghum bicolor Sudanense, Saccharum officinarum, Helianthus annuus, Crotalaria juncea, Citrus limetta, lopmoea batatas, Citrus reticulata, Xanthosoma sagittifolium, Manihot esculenta, Colocasia esculenta, Camellia sinensis, Eragrostis abyssinica, Phleum pratense, Nicotiana tabacum, Lycopersicum esculentum, Lotus spp., Aleurites spp., Brassica rapa, Urena lobate, Vanilla planifolia, Vicia sativa, Juglans spp., Citrullus lanatus, Acacia mearnsii, Triticum spp., Hordeum spp., Dioscorea spp.,* and *Ilex paraguariensis.*

Suitably, the plant or part thereof which may be protected by the compounds, compositions, or agents of the present disclosure is selected from a plant or part thereof which suffers from hemipteran, dipteran, coleopteran, blattodean and/or lepidopteran insect infestations, or which attracts hemipteran, dipteran, coleopteran, blattodean and/or lepidopteran insects. Suitably, the plant or part thereof which suffers from hemipteran, dipteran, coleopteran, blattodean and/or lepidopteran insect infestations, or which attracts hemipteran, dipteran, coleopteran, blattodean and/or lepidopteran insects is any of those listed above.

Suitably, the plant or part thereof which suffers from hemipteran insect infestations, or which attracts hemipteran insects, is any of those listed above.

Suitably, the plant or part thereof which suffers from dipteran insect infestations, or which attracts dipteran insects, is any of those listed above.

Suitably, the plant or part thereof which suffers from lepidopteran insect infestations, or which attracts lepidopteran insects, is any of those listed above.

Suitably, the plant or part thereof which suffers from coleopteran insect infestations, or which attracts coleopteran insects, is any of those listed above.

Suitably, the plant or part thereof which suffers from blattodean insect infestations, or which attracts blattodean insects, is any of those listed above.

In one example, the plant or part thereof is selected from a plant or part thereof which suffers from or attracts hemipteran insect infestations, for example: cereal crops such as wheat (*Triticum spp*.), oats (*Avena spp*)*,* rye (*Secale spp*.), barley (*Hordeum spp*.), rice (*Oryza spp*.) and corn (*Zea spp*.); fruit and vegetable crops including apples (*Malus spp*); pears (*Pyrus spp*); strawberry (*Fragaria spp*.), blueberry (*Vaccinum spp*.), blackberry (*Rubus spp*.), raspberry (*Rubus spp*.), citrus (*Citrus spp*.), olive (*Olea spp*.), durian (*Durio spp*.), longan (*Dimocarpus spp*.), litchi (*L. chinensis*), persimmon (*Diospyros spp*.); beans and peas (including but not limited to *Phaseolus, Vigna, Pisum, Lens, Glycine, Cicer, Cajanus, Arachis spp*)*,* sugar beet (*Beta vulgaris*)*,* sugar cane (*Saccharum spp*.), lettuce (*Lactuca spp*.), brassicas (*Brassica spp*.) including oil seed rape, alliums (*Allium spp*.), tomato (*Solanum spp*.), pepper (*Capsicum spp*.), asparagus (*A. officinalis*), melon, squash, pumpkins (*Cucumis spp*.), and tubers (potato) (*Solanum spp*.), or a part thereof.

In one example, the plant or part thereof is selected from a plant or part thereof which suffers from or attracts aphid insect infestations, suitably *M. persicae* insect infestations, including *Solanaceae, Cruciferae,* and *Leguminosae* for example: cereal crops such as wheat (*Triticum spp* including winter wheat *Triticum aestivum L*); fruit and vegetable crops including peach (*Prunus spp*.), strawberry (*Fragaria spp*.), blueberry (*Vaccinum spp.*), blackberry (*Rubus spp.*), raspberry (*Rubus spp*.), brassicas (*Brassica spp*.) such as oil seed rape, lettuce (*Lactuca spp*.), tomato (*Solanum spp*.), pepper (*Capsicum spp*.), beans and peas (including but not limited to *Vigna, Pisum spp*), melon, squash, pumpkins (*Cucumis spp*.), citrus (*Citrus spp*.), and tubers (potato) (*Solanum spp*.), or a part thereof. In one example, the plant is a vegetable crop, suitably a brassica spp.

In one example, the plant or part thereof is selected from a plant or part thereof which suffers from or attracts dipteran insect infestations, for example: cereals (*Triticum spp*.); oats (*Avena spp*);*,* rye (*Secale spp*.); barley (*Hordeum spp*,) rice (*Oryza spp*.) and corn (Zea *spp*.); beans and peas (including but not limited to *Phaseolus, Vigna, Pisum, Lens, Glycine, Cicer, Cajanus, Arachis spp*); fruit crops including apples (*Malus spp*), pears (*Pyrus spp*), strawberry (*Fragaria spp*.), blueberry (*Vaccinum spp*.), blackberry (*Rubus spp*.)*,* raspberry (*Rubus spp*.), cherry, plum, apricot, peach, nectarine (*Prunus spp*.), blackcurrant, redcurrant, whitecurrant, gooseberry (Ribes spp.), kiwi fruit (*Actinidia* spp), papaya (*Carica spp*.), avocado (*Persea spp*.), mango (*Mangifera indica L*)*,* longan (*Dimocarpus spp*.)*,* litchi (*L. chinensis*)*,* grapes (*Vitis spp*.), fig (*Ficus spp*.), passionfruit (*Passiflora spp*.), Asian pears (*Pyrus spp*), citrus (*Citrus spp*.), and olive (*Olea spp*.); vegetable crops including alliums (*Allium spp*.), aubergine, tomato (*Solanum spp*.) and peppers (*Capsicum spp*.)*,* lettuce (*Lactuca spp*.), brassicas (*Brassica spp*.) and courgette, melon, squash, pumpkins (*Cucumis spp*.); Apiaceae root crops including carrot (*Daucus spp*.), parsnip (*Pastinaca* spp.), or a part thereof.

In one example, the plant or part thereof is selected from a plant of part thereof which suffers from or attracts lepidoptera insect infestations, for example: cereal crops such as wheat (*Triticum spp*.)*,* oats (*Avena spp*), rye (*Secale spp*.)*,* barley (*Hordeum spp*.), rice (*Oryza spp*.) and corn (*Zea spp*.); fruit and vegetable crops including apples (*Malus spp*); pears (*Pyrus spp*); tree nuts (including for example almonds (*P. amygdalus*)*,* pistachio (*Pistacia vera*), walnuts (*Juglandaceae*)*,* hazlenuts (*Corylus*)); avocado, including Persea Americana (Lauraceaea), blueberry (*Vaccinum spp*.), citrus (*Citrus spp*.), olive (*Olea spp*.), durian (*Durio spp*.), longan (*Dimocarpus spp*.), litchi (*L. chinensis*), persimmon (*Diospyros spp*.); beans and peas (including but not limited to *Phaseolus, Vigna, Pisum, Lens, Glycine, Cicer, Cajanus, Arachis spp*), sugar beet (*Beta vulgaris*), sugar cane (*Saccharum spp*.),lettuce (*Lactuca spp*.), brassicas (*Brassica spp*.) including oil seed rape, alliums (*Allium spp*.), tomato (*Solanum spp*.), pepper (*Capsicum spp*.), asparagus (*A. officinalis*), melon, squash, pumpkins (*Cucumis spp*.), and tubers (potato) (*Solanum spp*.), or a part thereof.

### Insects

The compounds, compositions, and agents of the disclosure suitably have activity against insects as described above.

The compounds, compositions, and agents of the disclosure may be effective against any insects. Such as Arthropoda, in particular from the class of the arachnids, for example *Acarus spp., Aceria sheldoni, Aculops spp., Aculus spp., Amblyomma spp., Amphitetranychus viennensis, Argas spp., Boophilus spp., Brevipalpus spp., Bryobia praetiosa, Centruroides spp., Chorioptes spp., Dermanyssus gallinae, Dermatophagoides pteronyssius, Dermatophagoides farinae, Dermacentor spp., Eotetranychus spp., Epitrimerus pyri, Eutetranychus spp., Eriophyes spp., Halotydeus destructor, Hemitarsonemus spp., Hyalomma spp., Ixodes spp., Latrodectus spp., Loxosceles spp., Metatetranychus spp., Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vaejovis spp., Vasates lycopersici.*

Still other examples are from the order of the Anoplura (Phthiraptera), for example, *Damalinia spp., Haematopinus spp., Linognathus spp., Pediculus spp., Ptirus pubis, Trichodectes spp.*

Still other examples are from the order of the Chilopoda, for example, *Geophilus spp., Scutigera spp.*

Still other examples are from the order of the Coleoptera, for example, *Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Prostephanus truncatus, Psylliodes spp., Ptinus spp., Rhizobius ventralis, Rhizopertha dominica, Sitophilus spp., Sphenophorus spp., Stegobium paniceum, Sternechus spp., Symphyletes spp., Tanymecus spp., Tenebrio molitor, Tribolium spp., Trogoderma spp., Tychius spp., Xylotrechus spp., Zabrus spp.* Suitably compounds, compositions, and agents of the disclosure have activity against Coleoptera species selected from, for example: Bruchid beetle (*Bruchus rufimanus*), cabbage seed weevil (*Ceutorhynchus obstrictus*), cabbage stem flea beetle (*Psylliodes chrysocephalus*)*,* cabbage stem weevil (*Ceutorhynchus pallidactylus*), Chafer grubs (*Melolontha melolontha*)*,* Colorado potato beetle (*Leptinotarsa decemlineata*), pea and bean weevils (*Sitona lineatus*), pollen beetle (*Meligethes spp*.), pygmy beetle (*Atomaria linearis*), rape winter stem weevil (*Ceutorhynchus picitarsis*), and wireworms (*Agriotes spp*.)

Still other examples are from the order of the Collembola, for example, *Onychiurus armatus.*

Still other examples are from the order of the Diplopoda, for example, *Blaniulus guttulatus.*

Still other examples are from the order of the Diptera, for example, *Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Chrysops spp., Cochliomyia spp., Contarinia spp., Cordylobia anthropophaga, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hylemyia spp., Hyppobosca spp., Hypoderma spp., Linomyza spp., Lucilia spp., Lutzomia spp., Mansonia spp., Musca spp., Nezara spp., Oestrus spp., Oscinella frit, Pegomyia spp., Phlebotomus spp., Phorbia spp., Phormia spp., Prodiplosis spp., Psila rosae, Rhagoletis spp., Sarcophaga spp., Simulium spp., Stomoxys spp., Tabanus spp., Tannia spp., Tetanops spp., Tipula spp. Still other examples are from the order of the Heteroptera, for example, Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp. Still other examples are from the order of the Homoptera, for example, Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma pin, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp.*

Still other examples are from the order of the Hymenoptera, for example, *Acromyrmex spp., Athalia spp., Atta spp., Diprion spp., Hoplocampa spp., Lasius spp., Monomorium pharaonis, Solenopsis invicta, Tapinoma spp., Vespa spp.*

Still other examples are from the order of the Isopoda, for example, *Armadillidium vulgare, Oniscus asellus, Porcellio scaber.*

Still other examples are from the order of the Isoptera, for example, *Coptotermes spp., Cornitermes cumulans, Cryptotermes spp., Incisitermes spp., Microtermes obesi, Odontotermes spp., Reticulitermes spp.*

Still other examples are from the order of the Lepidoptera, for example, *Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Chematobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mods spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichophaga tapetzella, Trichoplusia spp., Tuta absoluta, Virachola spp.*

Still other examples are from the order of the Orthoptera, for example, *Acheta domesticus, Dichroplus spp., Gryllotalpa spp., Leucophaea maderae, Locusta spp., Melanoplus spp., Pulex irritans, Schistocerca gregaria.*

Still other examples are from the order of the Blattodea, for example, *Blatta orientalis, Blattella germanica, Periplaneta america, Periplaneta spp., Supella longipalpa,* termites of the infraorder Isoptera such as those of the family Termitidae.

Still other examples are from the order of the Siphonaptera, for example, *Ceratophyllus spp., Ctenocephalides spp., Tunga penetrans, Xenopsylla cheopis.*

Still other examples are from the order of the Symphyla, for example, *Scutigerella spp.*

Still other examples are from the order of the Thysanoptera, for example, *Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp.* Still other examples are from the order of the Zygentoma (=Thysanura), for example, *Lepisma saccharina, Thermobia domestica.* for example *Lepisma saccharina, Thermobia domestica.*

In one example, the compounds, compositions, and agents of the disclosure suitably have activity against insects of the order hemiptera, diptera, coleoptera, blattodea and/or lepidoptera. In one example, the compounds, compositions, and agents of the disclosure suitably have activity against insects of the order Lepidoptera. In one example, the compounds, compositions, and agents of the disclosure suitably have activity against moths. In one example, the compounds, compositions, and agents of the disclosure suitably have activity against the moth species *Plutella xylostella.* In one example, the compounds, compositions, and agents of the disclosure suitably have activity against the moth species *Spodoptera frugiperda.*

### Hemipteran insects

The compounds, compositions and agents of the disclosure suitably have activity against insects of the order Hemiptera, which comprises groups including aphids, planthoppers, leafhoppers, stink bugs, shield bugs and cicadas. Suitably, the compounds, compositions and agents of the disclosure suitably have activity against aphids.

Hemipterans are defined by distinctive mouthparts in the form of a "beak", comprising modified mandibles and maxillae which form a "stylet", sheathed within a modified labium.

Many insects within these groups have endogenous neuropeptides with sequence homology to the peptides described herein, suggesting that the compounds of the present disclosure may have activity against those insects.

The insects may belong to the sub-order *Sternorrhyncha,* e.g. to the superfamily of *Aphidoidea* (aphid superfamily), *Aleyrodoidea* (whiteflies), *Coccoidea* (scale insects), *Phylloxeroidea* (including *Phylloxeridae* or "phylloxerans", and *Adelgidae* or woolly conifer aphids) or *Psylloidea* (jumping plant lice etc.).

Thus, the insects may be aphids, i.e. members of the aphid superfamily (*Aphidoidea*)*.* Aphids (Hemiptera: *Aphididae*) are one of the most significant groups of agricultural pests and are vectors in the transmission of approximately 50% of all insect transmitted plant viruses. Within that superfamily, the aphids may be part of the family Aphididae, which contains sub-families *Aiceoninae, Anoeciinae, Aphidinae, Baltichaitophorinae, Calaphidinae, Chaitophorinae, Drepanosiphinae, Eriosomatinae, Greenideinae, Hormaphidinae, Israelaphidinae, Lachninae, Lizeriinae, Macropodaphidinae, Mindarinae, Neophyllaphidinae, Phloeomyzinae, Phyllaphidinae, Pterastheniinae, Saltusaphidinae, Spicaphidinae, Taiwanaphidinae, Tamaliinae* and *Thelaxinae.*

The aphids may, for example, be of the genus Acyrthosiphon (e.g. *Acyrthosiphon pisum*)*,* Aphis (e.g. *Aphis gossypii, Aphis glycines, Aphis fabae*), Diuraphis (e.g. *Diuraphis noxia*) Macrosiphum (e.g. *Macrosiphum rosae, Macrosiphum euphorbiae*), Myzus (e.g. *Myzus persicae*), Rhopalosiphum (e.g. *Rhopalosiphum padi*)*,* or Sitobion (e.g. *Sitobion avenae*).

*Myzus persicae* (peach potato aphid) is the most economically important aphid crop pest worldwide, with a global distribution and host range encompassing more than 400 species in 40 different plant families. For example, it is a major pest of agricultural crops including fruit and potatoes, and act as a vector for viruses.

*Macrosiphum rosae,* (rose aphid) is an important horticultural pest, especially of cultivated species of *Rosa,* and is a vector in the transmission of 12 plant viruses including the strawberry mild yellow edge virus.

*Aphis gossypii* (cotton or melon aphid) is a pest of Curcibitae and cotton.

Other than aphids, the insects may, for example, be of the Adelgidae family, e.g. of the genus Adelges (e.g. *Adelges tsugae*).

The insects may be of the Aleyrodidae family, e.g. of the genus Bemisia (e.g. *Bemisia tabaci*) or Trialeurodes (e.g. *Trialeurodes vaporariorum*).

The insects may be of the Psylloidea family, e.g. of the genus Pachypsylla (e.g. *Pachypsylla venusta*)*.*

As examples of hemipteran insects outside the sub-order Sternorryncha, the insects may be of the Cimicidae family, e.g. of the genus Cimex (bed bugs), e.g. *Cimex lectularius.*

The insects may be of the Cicadellidae family, e.g. of the genus Cuerna (e.g. *Cuerna arida*), Graminella (e.g. *Graminella nigrifrons*) or Homalodisca (e.g. *Homalodisca vitripennis*)*.* Also included in the Cicadellidae family are *Amrasca biguttula.*

The insects may be part of the Delphacidae family, e.g. of the genus Nilaparvata (e.g. *Nilaparvata lugens*) or Sogatella (e.g. *Sogatella furcifera*). For example, *Nilaparvata lugens* (brown planthopper) is a pest of rice crops, especially in Asia.

The insects may be of the Liviidae family, e.g. of the genus Diaphorina (e.g. *Diaphorina citri*).

The insects may be part of the Miridae family, e.g. of the genus Pseudatomoscelis (e.g. *Pseudatomoscelis seriatus*), Lygus (e.g. *Lygus hesperus*) or Tupiocoris (e.g. *Tupiocoris notatus*)*.* For example, *Pseudatomoscelis seriatus* (cotton fleahopper) is a pest of cotton.

The insects may be of the Pentatomidae family, e.g. of the genus Acrosternum (e.g. *Acrosternum hilare*), Banasa (e.g. *Banasa dimiata*), Euschistus (e.g. *Euschistus servus, Euschistus heroes*)*,* Halyomorpha (e.g. *Halyomorpha halys*)*,* Murgantia (e.g. *Murgantia histrionica*), Nezara (e.g. *Nezara viridula*), Plautia (e.g. *Plautia stali*), or Podisus (e.g. *Podisus maculiventris*). For example, *Acrosternum hilare* (green stink bug) is a significant pest of cotton. *Euschistus servus* (brown stink bug) is a pest of many agricultural crops including seeds, grains, nuts and fruits, especially in the southern USA. *Nezara viridula* is a pest of grain and soybean crops, especially in Brazil.

The insects may be of the Pyrrhocoridae family, e.g. of the genus Pyrrhocoris (e.g. *Pyrrhocoris apterus*).

The insects may be of the Reduviidae family, e.g. of the genus Rhodnius (e.g. *Rhodnius prolixus*), or Triatoma (e.g. *Triatoma infestans*). *Rhodnius prolixus* is a vector of human disease (Chagas disease).

The insects may be of the Triozidae family, e.g. of the genus Acanthocasuarina (e.g. *Acanthocasuarina muellerianae*).

In one example, the insect may be selected from the following species: H. *halys, E. heroes, A. hilare, A .gossypii, E. servus, M. persicae, N. viridula, N. lugens, P. seriatus,* and *R. prolixus.*

In one example, the insect is of the species *M. persicae.*

### Dipteran insects

The compounds, compositions and agents of the disclosure may have activity against insects of the order Diptera.

In particular, they may have activity against insects of the family Drosophilidae, such as fruit flies, including those of genus Drosophila, such as *Drosophila suzukii.* They may also have activity against insects of the family Tephritidae, including those of the genera *Anastrepha* (*Anastrepha* spp.); *Bactrocera* (*Bactrocera* spp.); *Ceratitis* (*Ceratitis* spp.); *Dacus* (*Dacus* spp.); *Rhagoletis* (*Rhagoletis* spp.); *Tephritis* (*Tephritis* spp.).

The families Drosophilidae and Tephritidae together are commonly referred to as fruit flies.

The compounds , compositions, and agents may also have activity against other important dipteran pests, such as flies of the family Chloropidae (chloropid flies) and those of the genera:
*Phytomyza (e.g. Phytomyza angelicastri);*
*Melani (e.g. Melani agromyza);*
*Antherigona (e.g. Antherigona* spp);
*Delia (e.g. Delia radicum);*
*Contarinia (e.g. Contarinia sorghicola);*

For more detail on these, and other examples, see *Developing the Arsenal Against Pest and Vector Dipterans: Inputs of Transgenic and Paratransgenic Biotechnologies, Ogaugwu and Durvasula, IntechOpen, 2017: DOI:10.5772*/*66440*

### Lepidopteran Insects

The compounds, compositions and agents of the disclosure may have activity against insects of the order Lepidoptera.

In particular, they may have activity against insects of the genera: *Heliothis, Plutella, Spodoptera, and Cydia.* Suitably the compounds, compositions and agents of the disclosure may have activity against the species: *Heliothis peltigera, H. virescens, Spodoptera spp.,* and *Cydia pomonella* (Codling Moth), *Larvae of Heliothis spp., including peltigera and virescens Spodoptera littoralis* (which represent a large variety of *Heliothinae* and *Spodoptera* moth species and are world-wide agricultural pests), *and Plutella xylostella* (diamondback moth, most important world-wide pest of Brassicas).

In one example, the compounds, compositions and agents of the disclosure may have activity against *Plutella xylostella.* In one example, the compounds, compositions and agents of the disclosure may have activity against *Spodoptera frugiperda.*

### Coleopteran Insects

The compounds, compositions and agents of the disclosure may have activity against insects of the order Coleoptera.

In particular, they may have activity against insects of the genera: *Bruchus, Ceutorhynchus, Psylliodes, Leptinotarsa, Sitona, Meligethes, Atomaria,* and *Agriotes.* Suitably the compounds, compositions and agents of the disclosure may have activity against the species: Bruchid beetle (*Bruchus rufimanus*)*,* cabbage seed weevil (*Ceutorhynchus obstrictus*), cabbage stem flea beetle (*Psylliodes chrysocephalus*), cabbage stem weevil (*Ceutorhynchus pallidactylus*), Chafer grubs (*Melolontha melolontha*), Colorado potato beetle (*Leptinotarsa decemlineata*)*,* pea and bean weevils (*Sitona lineatus*)*,* pollen beetle (*Meligethes spp*.), pygmy beetle (*Atomaria linearis*), rape winter stem weevil (*Ceutorhynchus picitarsis*), and wireworms (*Agriotes spp*.)

### Blattodean Insects

The compounds, compositions and agents of the disclosure may have activity against insects of the order Blattodea.

In particular, they may have activity against insects of the infraorder Isoptera. Suitably the compounds, compositions and agents of the disclosure may have activity against the species of the family Termitidae.

Suitably the compounds, compositions and agents of the disclosure may have activity against the species: *Blatta orientalis, Blattella germanica, Periplaneta america, Periplaneta* spp., *Supella longipalpa.*

### Household Pests

The compounds, compositions, and agents of the disclosure may also have activity against domestic pests, such as cockroaches and termites (such as those of the family Termitidae). Of more than 3000 species, these may include the German cockroach (*Blattella germanica*), the Oriental cockroach (*Blatta orientalis*), the American cockroach (*Periplaneta americana*) and the brown banded cockroach (*Supella longipalpa*). Cockroaches are common domestic pests worldwide, and may carry various diseases. Control of these and other domestic pests (e.g. ants) is envisaged by treating surfaces where the insects run, but particularly with food baits containing compounds, compositions and agents of the disclosure, and with direct spray application of compounds, compositions and agents of the disclosure.

Therefore further described herein is a bait comprising a compound or composition of the disclosure, suitably which may be a bait for domestic pests. Further described herein is a method of controlling domestic pests, reducing domestic pest populations, inhibiting domestic pest populations, increasing domestic pest mortality, the method comprising treating a surface (e.g. wood) which is contacted by the domestic pest with a compound or composition of the disclosure, or contacting the domestic pest with a compound or composition of the disclosure. Further details of such methods are described hereinbelow. Optionally treating or contacting may comprise a suitable means of application as described elsewhere herein, such as by spraying. Suitably therefore a sprayable formulation comprising a compound or composition of the disclosure is also envisaged. Suitable formulations are described elsewhere herein.

### Methods and Uses

### Methods of Increasing Insect Mortality

Further described herein is a method of increasing insect mortality, comprising contacting an insect or insect population with a compound, composition or combinations as described herein. The insect or insect population may be hemipteran, dipteran, coleopteran, blattodean, and/or lepidopteran insects.

In one example, there is provided a method of increasing dipteran insect mortality, comprising contacting a dipteran insect or dipteran insect population with a compound or composition or combinations of the disclosure.

In one particular example, there is provided a method of increasing *Drosophila suzukii* mortality, comprising contacting a *Drosophila suzukii* insect or insect population with a compound or composition or combinations of the disclosure.

In one example, there is provided a method of increasing hemipteran insect mortality, comprising contacting a hemipteran insect or hemipteran insect population with a compound or composition or combinations as defined herein.

In one particular example, there is provided a method of increasing aphid mortality, comprising contacting an aphid insect or insect population with a compound or composition or combinations as defined herein.

In one particular example, there is provided a method of increasing *Aphis fabae, Aphis gossypii, Acyrthosiphon pisum, Myzus persicae, Amrasca biguttula or Rhopalosiphum padi* mortality, comprising contacting an *Aphis fabae, Aphis gossypii, Acyrthosiphon pisum, Myzus persicae, Amrasca biguttula or Rhopalosiphum padi* insect or insect population with a compound or composition or combinations as defined herein.

In one particular example, there is provided a method of increasing *Myzus persicae* mortality, comprising contacting a *Myzus persicae* insect or insect population with a compound or composition or combinations as defined herein.

In one particular example, there is provided a method of increasing *Rhopalosiphum padi* mortality, comprising contacting a *Rhopalosiphum padi* insect or insect population with a compound or composition or combinations as defined herein.

In one example, there is provided a method of increasing lepidoptera insect mortality, comprising contacting a lepidopteran insect or lepidopteran insect population with a compound or composition or combinations as defined herein.

In one particular example, there is provided a method of increasing *Plutella xylostella* mortality, comprising contacting a *Plutella xylostella* insect or insect population with a compound or composition or combinations as defined herein.

In one particular example, there is provided a method of increasing *Spodoptera frugiperda* mortality, comprising contacting a *Spodoptera frugiperda* insect or insect population with a compound or composition or combinations as defined herein.

In one example, there is provided a method of increasing coleopteran insect mortality, comprising contacting a coleopteran insect or coleopteran insect population with a compound or composition or combinations as defined herein.

In one example, there is provided a method of increasing blattodean insect mortality, comprising contacting a blattodean insect or blattodean insect population with a compound or composition or combinations as defined herein.

### Methods of Inhibiting or Reducing infestation of a Plant or Site

Further described herein is a method of inhibiting infestation of a plant or plant part by insects, suitably by hemipteran, dipteran, coleopteran, blattodean and/or lepidopteran insects comprising contacting the plant or plant part with a compound, composition or combinations as described.

In one example, there is provided a method of inhibiting infestation of a plant by dipteran insects comprising contacting the plant with a compound or composition or combinations of the disclosure.

In one particular example, there is provided a method of inhibiting infestation of a plant by *Drosophila suzukii* comprising contacting the plant with a compound or composition or combinations of the disclosure.

In one example, there is provided a method of inhibiting infestation of a plant by hemipteran insects comprising contacting the plant with a compound or composition or combinations as defined herein.

In one example, there is provided a method of inhibiting infestation of a plant by aphids comprising contacting the plant with a compound or composition or combinations as defined herein.

In one example, there is provided a method of inhibiting infestation of a plant *by Aphis fabae, Aphis gossypii, Acyrthosiphon pisum, Myzus persicae or Rhopalosiphum padi* comprising contacting the plant with a compound or composition or combinations as defined herein.

In one particular example, there is provided a method of inhibiting infestation of a plant by *Rhopalosiphum padi* comprising contacting the plant with a compound or composition or combinations as defined herein.

In one example, there is provided a method of inhibiting infestation of a plant by *Myzus persicae* comprising contacting the plant with a compound or composition or combinations as defined herein.

In one example, there is provided a method of inhibiting infestation of a plant by *Halyomorpha halys* comprising contacting the plant with a compound or composition or combinations as defined herein.

In one example, there is provided a method of inhibiting infestation of a plant by lepidopteran insects comprising contacting the plant with a compound or composition or combinations as defined herein.

In one example, there is provided a method of inhibiting infestation of a plant *by Plutella xylostella* comprising contacting the plant with a compound or composition or combinations as defined herein.

In one example, there is provided a method of inhibiting infestation of a plant by *Spodoptera frugiperda* comprising contacting the plant with a compound or composition or combinations as defined herein.

In one example, there is provided a method of inhibiting infestation of a plant by coleopteran insects comprising contacting the plant with a compound or composition or combinations as defined herein.

In one example, there is provided a method of inhibiting infestation of a plant by blattodean insects comprising contacting the plant with a compound or composition or combinations as defined herein.

Further described herein is a method of inhibiting infestation of a site on which plants are growing or intended to be grown by insects, suitably by hemipteran, dipteran, coleopteran, blattodean and/or lepidopteran insects comprising contacting the site with a compound, composition, or combinations as described.

The method may be prophylactic. Thus, for example, the compound may be applied to the plant or plant part, or site, while the plant or part or field is free or substantially free of insects.

Suitably the site may be any agricultural site suitable for growing plants. Suitably the site may be any area or locale which may be suitable for growing plants, or in which plants are grown. Suitable sites may include farmland, brownfield sites, fields, greenhouses, conservatories, containers, aquaponics and hydroponics systems etc. In one example, the site is a field.

Alternatively, the plant or plant part or site may already be colonised or infested by insects, suitably by hemipteran, dipteran, coleopteran, blattodean and/or lepidopteran insects.

Further described herein is a method of reducing insect infestation of a plant or plant part, or of reducing insect load on a plant or plant part, the method comprising contacting the plant or plant part with a compound, composition, or combinations as described herein. Further described herein is also a method of reducing insect infestation of a field, or of reducing an insect load in a field, the method comprising contacting the field with a compound, composition, or combinations as described herein. Suitably of hemipteran, dipteran, coleopteran, blattodean and/or lepidopteran insects.

### Methods of Decreasing Insect Feeding

Further described herein is a method of decreasing insect feeding, comprising contacting an insect or insect population with a compound, composition or combinations as described herein. Suitably decreasing insect feeding on a plant or plant part. The insect or insect population may be hemipteran, dipteran, coleopteran, blattodean and/or lepidopteran insects.

### Methods of Protecting a Plant from Insects

Further described herein is a method of protecting a plant or part thereof from insects, or from insect infestation, and specifically a method of protecting a plant or plant part against hemipteran, dipteran, coleopteran, blattodean and/or lepidopteran insects or infestation thereof, wherein the method comprises the step of applying directly or indirectly to the plant or to a part of the plant, an insecticidal compound or composition, or combinations of the disclosure. Suitably applying indirectly may comprise applying the compound or composition or combinations of the disclosure to the field or locale in which the plant is growing, or in which it is intended to be grown.

Further described herein is post-harvest treatment methods for protecting or treating a harvested plant or a harvested part of the plant against insects, or insect infestation, specifically against hemipteran, dipteran, coleopteran, blattodean and/or lepidopteran insects or infestation thereof, the method comprising the step of applying directly or indirectly to the harvested plant or to a harvested part of the plant, an insecticidal compound or composition or combinations of the disclosure, under conditions effective to protect or treat the harvested plant or a harvested part of the plant against the insects. Suitably applying indirectly may comprise applying the compound or composition or combinations of the disclosure to the location in which the plant or a harvested part of the plant is stored, or in which it is intended to be stored.

Suitably the insect may be of the order hemiptera, diptera, coleoptera, blattodea and/or lepidoptera.

### Methods of Contacting an Insect

In one example, there is provided a method of contacting an insect with a compound, preferably an insect control agent, even more preferably an insecticide, said method comprising applying to or on sites frequented by the insect a compound or composition or combinations according to the disclosure.

The site, frequented by insects may be a natural habitat for insects, or a place regularly visited by insects. This site can be treated then with the compound or composition as described above: as a non-limiting example mosquito nets, impregnated with encapsulated insecticide, can be used as application method. Alternatively, said site is created by application of a visual lure or of an attractant for the insects. Visual lures are known to the person skilled in the art, and include but are not limited to light sources, coloured object and shapes or silhouettes that stand out of a contrasting background. As mentioned above, insect attractants include but are not limited to pheromones, kairomones and allomones. The attractant may be present in the composition or it may be applied separately from the compound or composition or combinations, to ensure that the insects are attracted to the site where the compound or composition or combinations is applied.

Suitably the insect may be of the order hemiptera, diptera, coleoptera, blattodea and/or lepidoptera. More suitably, the insect is of the order lepidoptera. Most suitably, the insect is a moth, such as *Plutella xylostella* or *Spodoptera frugiperda.*

### Application of the Compounds or Compositions

The compound or composition or combinations of the disclosure may be contacted with the insect or insect population, suitably this may comprise applying the compound or composition or combinations directly to an insect or insect population. For example, it may be applied topically. Alternatively, the compound or composition or combinations may be applied indirectly. For example, it may be applied to a substrate, site, or locale likely to come into contact with an insect or insect population. The substrate may be a plant or plant part, especially for Hemiptera or Diptera or Coleoptera or Lepidoptera or Blattodea which represent pests of plants (whether crops or horticultural plants), or may be a field, locale, or area suitably in which the plants are grown. Suitably therefore the compound or composition or combinations may be applied to the plant or plant part. Suitably therefore the compound or composition or combinations may be applied to the site in which the plant is grown.

However, for insects which represent pests to humans, such as the Cimicidae family (e.g. bedbugs of the genus Cimex, such as *Cimex lectularius*) or the Reduviidae family (e.g. of the genus Rhodnius such as *Rhodnius prolixus,* or Triatoma such as *Triatoma infestans*) which can be vectors of human disease, the substrate may be a domestic surface or article, such as bedding, a mattress, or any other suitable domestic surface. The compound or composition may be applied to the substrate in a form suitable for ingestion by an insect.

Suitably contacting may comprise feeding or spraying, for example. Suitably feeding may be encouraged via bait attractants, which may be comprised in a composition of the disclosure, as explained below.

The methods may comprise contacting or applying directly or indirectly to the plant or to a part of the plant a compound or composition or combinations as disclosed herein, for example at an application rate higher than 5g of the compound per hectare, such as but not limited to an application rate higher than 10g of the compound per hectare, such as an application rate higher than 24g of the compound per hectare, such as an application rate higher than 50g of the compound per hectare, such as an application rate higher than 75g of the compound per hectare, such as an application rate higher than 100g of the compound per hectare, or in particular an application rate higher than 200g of the compound per hectare. These methods may comprise applying directly or indirectly to the plant or to a part of the plant a compound, composition or combinations as disclosed herein, for example at an application rate between 5g and 100g of the compound composition or combinations per hectare, such as but not limited to an application rate of between 5g and 200g of the compound composition or combinations per hectare, in particular an application rate of between 5g and 50g of the compound composition or combinations per hectare, such as between 5g and 30g of the compound composition or combinations per hectare or between 10g and 25g per hectare.

The compounds or compositions or combinations as disclosed herein may be directly or indirectly contacted with/applied to the plant or to a part of the plant by spraying, atomizing, foaming, fogging, culturing in hydroculture, culturing in hydroponics, coating, submerging, injecting and/or encrusting, optionally post-harvest. Suitably contacting may comprise feeding or spraying, for example. In some examples, the contacting is by feeding. Contacting may comprise injecting a plant (e.g. a tree) or part thereof with the composition as defined herein, using systems and methods such as those described in WO2020/021041, WO2023/161802, WO2022/264053, WO2022/189386, WO2022/165248, WO2021/152093, WO2020/212612 and WO2020/021041.

Suitably the compound may be contacted with the insect or insect population, or plant or plant part, at any suitable concentration which is effective. Suitably the compositions and combinations may comprise such an effective concentration of a compound. Suitably the concentration of the compound is between 10⁻³ to 10⁻⁹ M, suitably between 10⁻⁴ to 10⁻⁶ M, suitably between 10⁻⁴ to 10⁻⁵ M.

### Use as a Plant Protection Agent

Further described herein is the use of a compound, composition or combination as described herein as a plant protection agent, and specifically for protecting a plant or plant part against insects, suitably against hemipteran, dipteran, coleopteran, blattodean and/or lepidopteran insects. Plant protection agents are described further hereinabove.

### Use as an Insect Control Agent

Further described herein is the use of a compound or composition or combination thereof as described herein as an insect control agent, specifically in methods of increasing mortality in insects, or a method of inhibiting infestation of a plant by insects.

Further described herein is the use of a compound or composition or combination thereof as described herein as an insect control agent, specifically in methods of increasing hemipteran, dipteran, coleopteran, blattodean and/or lepidopteran insect mortality, or in a method of inhibiting or reducing infestation, or reducing insect load of a plant by insects, suitably by hemipteran, dipteran, coleopteran, blattodean and/or lepidopteran insects.

Further described herein is the use of a compound or composition or combination thereof as described herein as an insect control agent, specifically by having a biostatic effect on hemipteran, dipteran, coleopteran, blattodean and/or lepidopteran insects, a biocidal effect on hemipteran, dipteran, coleopteran, blattodean and/or lepidopteran insects, and/or a pesticidal effect hemipteran, dipteran, coleopteran, blattodean and/or lepidopteran insects.

"Biostatic (effect)" or "biostatic use", as used herein, includes any effect or use of a compound or composition or combination as described herein (optionally comprised in a biostatic, biocidal, fungicidal or fungistatic composition as defined herein) for controlling, modulating or interfering with the harmful activity of a pest, such as a plant pest or a plant pathogen. Suitably the pest is of hemipteran, dipteran, coleopteran, blattodean and/or lepidopteran insect orders. Suitably including but not limited to inhibiting the growth or activity of the insect, altering the behaviour of the insect, and repelling or attracting the insect in plants, plant parts or in other agro-related settings, such as for example for household uses or in soil.

"Pesticidal activity" or "biocidal activity", as used interchangeably herein, means killing the pest or severely disabling the pest. Suitably which may be the same as insecticidal activity wherein the pest is an insect. Suitably, the compound, composition or combination may be for use as an insect control agent wherein the insect encodes an AKH peptide.

Suitably, the compound or composition or combination may be for use as an insect control agent wherein the insect is of the order diptera. Suitably, the compound may be for use as an insect control agent wherein the insect is of the genus *Drosophila.* Suitably, the compound may be for use as an insect control agent wherein the insect is *Drosophila suzukii.*

Suitably, the compound or composition or combination as defined herein may be for use as an insect control agent wherein the insect is of the order hemiptera. Suitably, the compound or composition or combination as defined herein may be for use as an insect control agent wherein the insect is an aphid. Suitably, the compound or composition or combination as defined herein may be for use as an insect control agent wherein the insect is of the genus *Myzus.* Suitably, the compound or composition or combination as defined herein may be for use as an insect control agent wherein the insect is *Myzus persicae.*

Suitably, the compound or composition or combination as defined herein may be for use as an insect control agent wherein the insect is of the order hemiptera. Suitably, the compound or composition or combination as defined herein may be for use as an insect control agent wherein the insect is of the genus *Halyomorpha.* Suitably, the compound or composition or combination as defined herein may be for use as an insect control agent wherein the insect is *Halyomorpha halys.*

Suitably, the compound or composition or combination as defined herein may be for use as an insect control agent wherein the insect is of the order lepidoptera. Suitably, the compound or composition or combination as defined herein may be for use as an insect control agent wherein the insect is of the genus *Plutella.* Suitably, the compound or composition or combination as defined herein may be for use as an insect control agent wherein the insect is *Plutella xylostella.*

Suitably, the compound or composition or combination as defined herein may be for use as an insect control agent wherein the insect is of the order lepidoptera. Suitably, the compound or composition or combination as defined herein may be for use as an insect control agent wherein the insect is of the genus *Spodoptera.* Suitably, the compound or composition or combination as defined herein may be for use as an insect control agent wherein the insect is *Spodoptera frugiperda.*

Suitably, the compound or composition or combination as defined herein may be for use as an insect control agent wherein the insect is of the order coleoptera.

Suitably, the compound or composition or combination as defined herein may be for use as an insect control agent wherein the insect is of the order blattodea. Suitably, the compound or composition or combination as defined herein may be for use as an insect control agent wherein the insect is a cockroach or a termite.

### Methods of controlling Insect Fecundity

Further described herein are insecticidal compounds, compositions and combinations as described herein for controlling fecundity of insect species. Suitably, the insect may be selected from hemipteran, dipteran, coleopteran, blattodean or lepidopteran insects, such as those described anywhere herein. Suitably wherein in some examples the hemipteran insect is *Myzus persicae.* Suitably wherein in some examples the dipteran insect is *D. suzukii.* Suitably wherein in some examples the lepidopteran insect is *Plutella xylostella.* Suitably wherein in some examples the lepidopteran insect is *Spodoptera frugiperda.*

Further described herein is a method of controlling fecundity of a hemipteran insect, the method comprising contacting a compound of the disclosure, or a composition of the disclosure to a substrate, e.g. a plant or soil. Suitably, the hemipteran insect is any of those disclosed herein, e.g., an aphid.

Further described herein is method of controlling fecundity of a dipteran insect, the method comprising contacting a compound of the disclosure, or a composition of the disclosure to a substrate, e.g. a plant or soil. Suitably, the dipteran insect is any of those disclosed herein.

Further described herein is a method of controlling fecundity of a lepidopteran insect, the method comprising contacting a compound of the disclosure, or a composition of the disclosure to a substrate, e.g. a plant or soil. Suitably, the lepidopteran insect is any of those disclosed herein.

Further described herein is a method of controlling fecundity of a coleopteran insect, the method comprising contacting a compound of the disclosure, or a composition of the disclosure to a substrate, e.g. a plant or soil. Suitably, the coleopteran insect is any of those disclosed herein.

Further described herein is a method of controlling fecundity of a blattodean insect, the method comprising contacting a compound of the disclosure, or a composition of the disclosure to a substrate, e.g. a plant or soil. Suitably, the blattodean insect is any of those disclosed herein.

Further described herein is a method of controlling fecundity of a hemipteran insect, the method comprising contacting a combination as described herein, to a substrate, e.g. a plant or soil. Suitably, the hemipteran insect is any of those disclosed herein. Suitably, the combination comprises a compound of the disclosure, and a kinin peptide, or an analogue thereof.

Further described herein is a method of controlling fecundity of a dipteran insect, the method comprising contacting a combination as described herein, to a substrate, e.g. a plant or soil. Suitably, the dipteran insect is any of those disclosed herein. Suitably, the combination comprises a compound of the disclosure, and a kinin peptide, or an analogue thereof.

Further described herein is a method of controlling fecundity of a lepidopteran insect, the method comprising contacting a combination as described herein, or a composition of the disclosure to a substrate, e.g. a plant or soil. Suitably, the lepidopteran insect is any of those disclosed herein. Suitably, the combination comprises a compound of the disclosure, and a kinin peptide, or an analogue thereof.

Further described herein is a method of controlling fecundity of a coleopteran insect, the method comprising contacting a combination as described herein, or a composition of the disclosure to a substrate, e.g. a plant or soil. Suitably, the coleopteran insect is any of those disclosed herein. Suitably, the combination comprises a compound of the disclosure, and a kinin peptide, or an analogue thereof.

Further described herein is a method of controlling fecundity of a blattodean insect, the method comprising contacting a combination as described herein, or a composition of the disclosure to a substrate, e.g. a plant or soil. Suitably, the blattodean insect is any of those disclosed herein. Suitably, the combination comprises a compound of the disclosure, and a kinin peptide, or an analogue thereof.

Further described herein is a method of controlling fecundity of a hemipteran insect or insect population, the method comprising contacting a compound of the disclosure, or a composition of the disclosure to a hemipteran insect or insect population. Suitably, the hemipteran insect is any of those disclosed herein.

Further described herein is a method of controlling fecundity of a dipteran insect or insect population, the method comprising contacting a compound of the disclosure, or a composition of the disclosure to a dipteran insect or insect population. Suitably, the dipteran insect is any of those disclosed herein.

Further described herein is a method of controlling fecundity of a lepidopteran insect or insect population, the method comprising contacting a compound of the disclosure, or a composition of the disclosure to a lepidopteran insect or insect population. Suitably, the lepidopteran insect is any of those disclosed herein.

Further described herein is a method of controlling fecundity of a coleopteran insect or insect population, the method comprising contacting a compound of the disclosure, or a composition of the disclosure to a coleopteran insect or insect population. Suitably, the coleopteran insect is any of those disclosed herein.

There is also provided a method of controlling fecundity of a blattodean insect or insect population, the method comprising contacting a compound of the disclosure, or a composition of the disclosure to a blattodean insect or insect population. Suitably, the blattodean insect is any of those disclosed herein.

Further described herein is a method of controlling fecundity of a hemipteran insect or insect population, the method comprising contacting a combination as described herein, to a hemipteran insect or insect population. Suitably, the hemipteran insect is any of those disclosed herein. Suitably, the combination comprises a compound of the disclosure, and a kinin peptide, or an analogue thereof.

Further described herein is a method of controlling fecundity of a dipteran insect or insect population, the method comprising contacting a combination as described herein, to a dipteran insect or insect population. Suitably, the dipteran insect is any of those disclosed herein. Suitably, the combination comprises a compound of the disclosure, and a kinin peptide, or an analogue thereof.

Further described herein is a method of controlling fecundity of a lepidopteran insect or insect population, the method comprising contacting a combination as described herein, or a composition of the disclosure to a lepidopteran insect or insect population. Suitably, the lepidopteran insect is any of those disclosed herein. Suitably, the combination comprises a compound of the disclosure, and a kinin peptide, or an analogue thereof.

Further described herein is a method of controlling fecundity of a coleopteran insect or insect population, the method comprising contacting a combination as described herein, or a composition of the disclosure to a coleopteran insect or insect population. Suitably, the coleopteran insect is any of those disclosed herein. Suitably, the combination comprises a compound of the disclosure, and a kinin peptide, or an analogue thereof.

Further described herein is a method of controlling fecundity of a blattodean insect or insect population, the method comprising contacting a combination as described herein, or a composition of the disclosure to a blattodean insect or insect population. Suitably, the blattodean insect is any of those disclosed herein. Suitably, the combination comprises a compound of the disclosure, and a kinin peptide, or an analogue thereof.

In the context of the present disclosure, "controlling fecundity" refers to reducing, inhibiting, or eliminating the presence of an insect species during one or more of its growth stages. For example, the compositions of the present disclosure may be used for controlling the growth of mites at any stage such as egg, larva, nymph, and adult form.

### Combinations with Further Insecticides

The compound of the disclosure may be used in combination with one or more further insecticides or insecticidal compounds, such as those as described herein. There is also provided a combination comprising a compound of the disclosure, or a salt or solvate thereof, in combination with one or more additional active insecticides, or insecticidal compounds.

The composition of the disclosure may further comprise one or more additional active insecticides, or active insecticidal compounds.

Suitably, the insecticide may be selected from an insect neuropeptide or analogues thereof such as a kinin peptide, an AKH peptide, a DH31 peptide, a DH44 peptide, a pyrokinin peptide or a CAPA peptide (e.g. a CAPA-1, CAPA-2 or CAPA-3 analogue). In a particular example, the further insecticide is a kinin peptide, for example SB-P-69 ([Hy]-NFSPWG-[NH₂], SEQ ID NO:14). Additionally or alternatively, the insecticide may be selected from a chemical insecticide, such as: Pyrethroids (Permethrin, Cypermethrin, Deltamethrin); Organophosphates (Malathion, Chlorpyrifos, Diazinon); Neonicotinoids (Imidacloprid, Clothianidin, Thiamethoxam); Carbamates (Carbaryl, Methomyl, Propoxur); Botanical Insecticides (Pyrethrin (derived from chrysanthemum flowers), Rotenone (derived from certain plant roots)); Biopesticides (Bacillus thuringiensis (Bt) products, Beauveria bassiana (fungus), Metarhizium anisopliae (fungus)); Insect Growth Regulators (IGRs) (Methoprene, Pyriproxyfen); Fipronil; Spinosad; Avermectins (including abamectin and ivermectin); Chitin Synthesis Inhibitors (Diflubenzuron, Hexaflumuron); Piperonyl Butoxide; Flonicamid; Indoxacarb; and Sulfoxaflor.

Suitably said further insecticides or insecticidal compounds may be comprised in a composition of the disclosure.

The methods of the disclosure may further comprise contacting the insect population or plant or part thereof with a further insecticide agent, or insecticidal compound, such as those described herein. Suitably, the further insecticide or insecticidal compound is a kinin peptide.

Suitably any references herein to the compound of the disclosure or a composition thereof may equally refer to a combination of a compound of the disclosure with one or more insecticides or insecticidal compounds, or a composition containing such a combination.

Suitably, there is provided a combination comprising a compound of the disclosure, or a salt or solvate thereof, in combination with one or more of the peptides listed in the table below:

| **Family** | **Peptide** | **Sequence** | **SEQ ID NO.** |
|---|---|---|---|
| **Pyrokinin** | SB-P-46 | [Hy]-SPPYSPPFSPRL-[NH₂] | 11 |
| | SB-P-47 | [Pyr]-AIMARPQVPRL-[NH₂] | 3 |
| | SB-P-48 | Hy-EQNVQSNGEPAYRVRTPRL-[NH₂] | 15 |
| | SB-P-49 | [Hy]-SVPFKPRL-[NH₂] | 4 |
| | SB-P-51 | [Hy]-LRQLQSNGEPAYRVRTPRL-[NH₂] | 5 |
| | SB-P-80 | [Hy]-NADEDQQQSVDFTPRL-[NH₂] | 6 |
| | SB-P-81 | [Hy]-GGSMTFSPRL-[NH₂] | 7 |
| **Kinin** | SB-P-70 | [Hy]-KVKFSAWG-[NH₂] | 8 |
| | SB-P-65 | [Hy]-RQKTVFSSWG-[NH₂] | 9 |
| | SB-P-66 | [Hy]-PAFSSWG-[NH₂] | 10 |
| | SB-P-69 | [Hy]-NFSPWG-[NH₂] | 14 |
| **AKH** | SB-P-39 | [Palm]-QLTFSPDW-[NH₂] | 12 |
| | SB-P-41 | Hy-QLTFSPDW-[NH₂] | 16 |
| | SB-P-42 | [Pyr]-LTFSPDW-[NH₂] | 13 |

In one example, the compound of the disclosure is utilised in combination with a pyrokinin peptide, e.g.

| **Family** | **Peptide** | **Sequence** | **SEQ ID NO.** |
|---|---|---|---|
| **Pyrokinin** | SB-P-46 | [Hy]-SPPYSPPFSPRL-[NH₂] | 11 |
| | SB-P-47 | [Pyr]-AIMARPQVPRL-[NH₂] | 3 |
| | SB-P-48 | Hy-EQNVQSNGEPAYRVRTPRL-[NH₂] | 15 |
| | SB-P-49 | [Hy]-SVPFKPRL-[NH₂] | 4 |
| | SB-P-51 | [Hy]-LRQLQSNGEPAYRVRTPRL-[NH₂] | 5 |
| | SB-P-80 | [Hy]-NADEDQQQSVDFTPRL-[NH₂] | 6 |
| | SB-P-81 | [Hy]-GGSMTFSPRL-[NH₂] | 7 |

In one example, the compound of the disclosure is utilised in combination with a kinin peptide, e.g.

| **Family** | **Peptide** | **Sequence** | **SEQ ID NO.** |
|---|---|---|---|
| **Kinin** | SB-P-70 | [Hy]-KVKFSAWG-[NH₂] | 8 |
| | SB-P-65 | [Hy]-RQKTVFSSWG-[NH₂] | 9 |
| | SB-P-66 | [Hy]-PAFSSWG-[NH₂] | 10 |
| | SB-P-69 | [Hy]-NFSPWG-[NH₂] | 14 |

In one example, the compound of the disclosure is utilised in combination with a CAPA peptide (e.g. a CAPA2 peptide).

In one example, the compound of the disclosure is utilised in combination with a further AKH peptide, e.g.

| **Family** | **Peptide** | **Sequence** | **SEQ ID NO.** |
|---|---|---|---|
| **AKH** | SB-P-39 | [Palm]-QLTFSPDW-[NH₂] | 12 |
| | SB-P-41 | Hy-QLTFSPDW-[NH₂] | 16 |
| | SB-P-42 | [Pyr]-LTFSPDW-[NH₂] | 13 |

Suitably any of the above combinations of compounds may be comprised within a composition of the disclosure.

The choice of ancillary or additional insecticides will typically depend on the particular target species.

### Beneficial Insect Species

The compounds and compositions and combinations of the disclosure may be substantially non-toxic to beneficial insect species, including species which prey on pests and pollinator species. Important pollinator species, such as insects of the superfamily Apoidea, including bees, such as the Apidae, e.g. those of the genus Bombus, such as *Bombus terrestris.* Important predatory species include Coccinellidae (lady bugs) such as *Adalia bipunctata.*

By substantially non-toxic, it is meant that the compounds and compositions and combinations of the disclosure do not cause death of the beneficial insect species (e.g. pollinator species), suitably that they do not cause premature death of the beneficial insect species (e.g. pollinator species). It is also meant that the compounds and compositions and combinations of the disclosure do not cause any detrimental side effects to the beneficial insect species (e.g. pollinator species), for example they do not have a negative effect on feeding behaviour, or ability to move.

### Compositions

The present inventors have provided compositions comprising at least one insecticidal compound or combination thereof of the disclosure, which can specifically bind to an insect. Importantly, through this interaction with a specific molecular structure of the insect, such as a receptor, suitably a neurological receptor, the compositions disclosed herein are capable of inhibiting, preventing or reducing one or more biological activities of the insect, such that the growth or fecundity of the insect is inhibited, prevented or reduced. In certain examples, the compositions as disclosed herein are capable of killing insects through the specific interaction of at least one insecticidal compound, which can specifically bind to an insect receptor, and which is comprised in the compositions.

Compositions of the disclosure, or for use in accordance with the disclosure, typically comprise a compound as described in combination with one or more ancillary component such as solvents, carriers, diluents, adjuvants, preservatives, dispersants, emulsifying agents, or synergists. Suitably the composition may be an agricultural composition, an insect control composition (e.g. insecticide composition), or a plant protection composition.

In any of these examples, the compound or combination of compounds of the disclosure may be provided as part of a composition, such as an agricultural composition, an insect control composition (e.g. insecticide composition) or a plant protection composition. Reference to application or use of a compound or combination herein should therefore be construed as encompassing application or use of a suitable composition thereof, unless the context demands otherwise.

The composition comprises a compound as described herein in admixture with one or more ancillary component such as solvents, carriers, diluents, adjuvants, preservatives, dispersants, emulsifying agents, or synergists.

The composition may further comprise a combination with one or more additional active insecticides as described herein.

Further described herein is a composition, e.g. an agricultural composition, an insect control composition or plant protection composition, comprising a compound of the disclosure, or a combination thereof, in admixture with one or more solvents, carriers, diluents, adjuvants, preservatives, dispersants, emulsifying agents, or synergists. The composition may be an aqueous composition. Further details of which are explained hereinbelow.

"Agricultural", as used herein, means suitable for use in the agricultural or agrochemical industry, including horticulture, floriculture and home and garden uses, but also products intended for non-crop related uses such as public health/pest control operator uses to control undesirable insects and rodents, household uses, such as household fungicides and insecticides and agents, for protecting plants or parts of plants, crops, bulbs, tubers, fruits (e.g. from harmful organisms, diseases or pests); for controlling, preferably promoting or increasing, the growth of plants; and/or for promoting the yield of plants, crops or the parts of plants that are harvested (e.g. its fruits, flowers, seeds etc.). Examples of such substances will be clear to the skilled person and preferably in the context of the present disclosure, include compounds that are active as insecticides (e.g. contact insecticides or systemic insecticides, including insecticides for household use). Other such agrochemicals may be pesticides, growth regulators, nutrients/fertilizers, repellants, defoliants etc.

"Agricultural use", as used herein, not only includes the use of the insecticidal compounds of the disclosure, combinations thereof or compositions, and optionally the agrochemicals as defined above (for example, pesticides, growth regulators, nutrients/fertilizers, repellants, defoliants etc.), that are suitable and/or intended for use in field grown crops (e.g., agriculture), but also includes the use of compounds of the disclosure combinations thereof or compositions and said agrochemicals as defined above that are meant for use in greenhouse grown crops (e.g. horticulture/floriculture) or hydroponic culture systems and even the use of compounds combinations thereof or compositions of the disclosure and agrochemicals as defined above that are suitable and/or intended for non-crop uses such as uses in private gardens, household uses (for example, herbicides or insecticides for household use), or uses by pest control operators (for example, weed control etc.).

Suitably an insect control composition according to the disclosure is for controlling insect populations. Formulations of such compositions for controlling insect populations are known to the person skilled in the art and include, but are not limited to liquid emulsifiable concentrates, wettable powders, solutions, suspension concentrates, emulsions, suspoemulsions, granules and water dispersible granules (Mulqueen, 2003). Preferably, said insect control compositions according to the disclosure comprise an insecticidal compound of the disclosure, and optionally a combination of further insecticidal compounds. Suitably said insecticidal compound is comprised in a carrier as described hereinbelow.

Compositions of the disclosure may comprise a pesticide formulation or an agrochemical formulation. A "pesticide formulation" as used herein means any composition comprising a compound or combination of compounds intended for preventing, destroying, repelling, attracting or mitigating any pest. An "agrochemical formulation" as used herein means a composition for agricultural use, comprising a biologically active agent, optionally with one or more additives favoring optimal dispersion, atomization, distribution, retention and/or activity of agrochemicals. As a non-limiting example such additives are diluents, solvents, adjuvants, surfactants, wetting agents, spreading agents, oils, stickers, penetrants, buffering agents, acidifiers, defoaming agents or drift control agents.

A composition as used herein means a composition comprising at least one active substance, suitably an insecticidal compound of the disclosure, optionally with one or more additives favouring optimal dispersion, atomization, deposition, leaf wetting, distribution, retention and/or uptake of said active substance. It will become clear from the further description herein that a composition as used herein includes biological insect control agents or biological insecticides, and these terms will be interchangeably used in the present application. Accordingly, a composition as used herein includes compositions comprising at least one biological molecule as an active ingredient, substance or principle for controlling pests in plants or in other agro-related settings (such for example in soil). Suitably wherein the at least one biological molecule comprises an insecticidal compound of the disclosure or a combination thereof. Suitably wherein the pest is an insect. As a non-limiting example, the additives in the compositions disclosed herein may include but are not limited to diluents, solvents, adjuvants, surfactants, wetting agents, spreading agents, oils, stickers, thickeners, penetrants, buffering agents, acidifiers, antisettling agents, anti-freeze agents, photoprotectors, defoaming agents, biocides and/or drift control agents.

Suitably, the compositions of the disclosure are aqueous compositions.

The compound content of the composition can vary within wide limits. The compound concentration of the composition is suitably an effective amount, and can be from 0.0000001 to 95% by weight of the compound, preferably between 0.0001 and 1% by weight. The terms "effective amount" and "effective dose", as used herein, mean the amount needed to achieve the desired result or results.

In a specific example the concentration of the compound of the disclosure contained in the composition may be at least 0.0001% by weight. In a specific example the concentration of the compound of the disclosure contained in the composition may be up to 50% by weight. In a specific example the concentration of the compound of the disclosure contained in the composition may be from 0.0001% to 50% by weight. In particular examples, the present disclosure provides compositions comprising at least one insecticidal compound of the disclosure, wherein the concentration of the at least one compound of the disclosure in the composition ranges from 0.001% to 50% by weight. In yet another specific example the concentration of the at least one compound of the disclosure contained in the composition may be from 0.001 % to 50% by weight. In yet another specific example the concentration of the at least one compound of the disclosure contained in the composition may be from 0.01% to 50% by weight. In yet another specific example the concentration of the at least one compound of the disclosure contained in the composition may be from 0.1% to 50% by weight.

The compositions of the disclosure, or for use in accordance with the disclosure, may comprise more than one compound of the disclosure in combination, and/or with other insecticidal compounds as described herein in combination. Therefore the compositions of the disclosure may comprise a first compound of the disclosure and a second compound of the disclosure, or a first compound of the disclosure and a second insecticidal compound, for example. Suitably the first and second compound may be any of those described herein, and may be present in the composition in any relative proportion.

The composition may be an aqueous composition, e.g. a saline composition. The aqueous composition may contain one or more buffers, such as a phosphate buffer (e.g. phosphate buffered saline) or a Tris buffer. Alternatively the composition may be an oil dispersion or an emulsion, e.g. an oil and water emulsion. Alternatively the composition may be a suspension, powder, foam, paste, granule, aerosol, impregnated natural and synthetic substance, or encapsulated in polymeric substance for example. A suitable form of the composition may be chosen for the intended use having regard to the target insect, and to its habitat.

Adjuvants may enhance product performance, for example, by increasing the efficiency of the delivery of active ingredients, reducing the level of active ingredient required, or extending the spectrum of effectiveness.

Different types of adjuvants offer various benefits and advantages, which are achieved by modulating properties such as spray formation, spray retention, wetting, deposit formation or uptake.

Adjuvants modulating spray formation may influence spray quality by reducing spray drift and wastage, allowing more of the product to reach the target. This can reduce use rates, leading to a better environmental profile and a potentially more cost effective solution. Such adjuvants include non-ionic surfactants and emulsifier blends.

Adjuvants modulating spray retention may dissipate the kinetic energy of the droplet during impact, meaning the likelihood of bounce or run-off is reduced. Such adjuvants include alkyl polyglucosides, alkoxylated alcohols, and polyoxyethylene monobranched alcohols (e.g. polyoxyethylene (8) monobranched alcohol).

Adjuvants modulating wetting properties (i.e. wetting agents) may reduce surface tension and contact angle, leading to enhanced coverage. Such adjuvants include polyoxyethylene sorbitan monolaurate (e.g. polyoxyethylene (8) sorbitan monolaurate), surfactant blends, and alkyl polyglucosides.

Adjuvants modulating deposit formation may influence evaporation of water from the droplet and thus provide a more homogeneous distribution. Such adjuvants include alkoxylated polyol esters, polyoxyethylene sorbitan monolaurate (e.g. polyoxyethylene (12) sorbitan monolaurate), and alkyl polyglucoside.

Adjuvants modulating uptake can improve penetration and uptake of active ingredients. e.g. through the insect cuticle, resulting in increased bioavailability. Such adjuvants include alkoxylated polyol esters and polyoxyethylene sorbitan monolaurate (e.g. polyoxyethylene (12) sorbitan monolaurate and polyoxyethylene (16) sorbitan monolaurate).

Dispersants may be aqueous or non-aqueous. An oil dispersion (OD) formulation typically comprises a solid active ingredient dispersed in oil. The oil can vary from paraffinic to aromatic solvent types and vegetable oil or methylated seed oils. Typically the active ingredient is uniformly suspended in the oil phase. Although primarily used for water sensitive active ingredients, OD formulations have extended to other active ingredients due to their better spray retention, spreading, foliar uptake, and penetration enhancement (e.g. across the insect cuticle) as the carrier oil often acts as an adjuvant.

Oils suitable for use in OD dispersions include linseed, rapeseed and soyabean oils.

Aqueous dispersants may be used, for example, to improve stability in the spray tank after dilution in water, and may include modified styrene acrylic polymers, and polymeric amphoteric dispersants and adjuvants.

An emulsifier may be employed to emulsify a continuous oil phase into water when an OD formulation is diluted prior to being sprayed. The emulsifier may be selected based upon its ability to spontaneously form the emulsion. Their performance is primarily dictated by the nature of the surfactant and their collective effect on how they arrange themselves at the oil/water interface. Examples include polyoxyethylene sorbitol hexaoleate (e.g. polyoxyethylene (40) sorbitol hexaoleate), emulsifier blends, and calcium alkylaryl sulphonate.

The composition may further comprise an adhesive or a dye.

The compound may be provided in the form of a concentrate, for dilution prior to application. Alternatively the compound may be provided in a solid form to be suspended or dissolved prior to formulation.

The compositions as disclosed herein are themselves in fairly diverse, solid or liquid, forms. As solid composition forms, there may be mentioned dustable powders (content of active substance which may be up to 100%) and granules, in particular those obtained by extrusion, by compacting, by impregnation of a granulated carrier, by granulation using a powder as starting material (the content of active substance in these granules being between 0.5 and 80% for these latter cases). Such solid compositions may be optionally used in the form of a liquid which is viscous to a greater or lesser degree, depending on the type of application desired, for example by diluting in water. As liquid composition forms or forms intended to constitute liquid compositions during application, there may be mentioned solutions, in particular water-soluble concentrates, emulsions, suspension concentrates, wettable powders (or spraying powder), oils and waxes. The suspension concentrates, which can be applied by spraying, are prepared so as to obtain a stable fluid product which does not form a deposit and they usually contain from 10 to 75% of active substance, from 0.5 to 15% of surfactants, from 0.1 to 10% of thixotropic agents, from 0 to 10% of appropriate additives, such as antifoams, corrosion inhibitors, stabilizers, penetrating agents and adhesives and, as carrier, water or an organic liquid in which the active substance is not or not very soluble: some organic solids or inorganic salts may be dissolved in the carrier to help prevent sedimentation or as antigels for water.

A "carrier", as used herein, means any solid, semi-solid or liquid carrier in or on(to) which an active substance, such as the insecticidal compounds or combinations of the disclosure, can be suitably incorporated, included, immobilized, adsorbed, absorbed, bound, encapsulated, embedded, attached, or comprised. Non-limiting examples of such carriers include nanocapsules, microcapsules, nanospheres, microspheres, nanoparticles, microparticles, liposomes, vesicles, beads, a gel, weak ionic resin particles, liposomes, cochleate delivery vehicles, small granules, granulates, nano-tubes, bucky-balls, water droplets that are part of an water-in-oil emulsion, oil droplets that are part of an oil-in-water emulsion, organic materials such as cork, wood or other plant-derived materials (e.g. in the form of seed shells, wood chips, pulp, spheres, beads, sheets or any other suitable form), paper or cardboard, inorganic materials such as talc, clay, microcrystalline cellulose, silica, alumina, silicates and zeolites, or even microbial cells (such as yeast cells) or suitable fractions or fragments thereof.

In one example, the carrier is a liposome. In one example, the composition of the disclosure comprises one more liposomes suitably wherein each liposome comprises a compound of the disclosure, or combination thereof. Suitably therefore the disclosure provides a composition or formulation comprising a plurality of liposomes, said liposomes comprising a compound of the disclosure, or a combination thereof. Suitably the liposomes comprise a lipid component, suitably which may be soy lecithin and glycerol. Suitably the liposomes are formed of soy lecithin and glycerol. Suitably the liposomes are formed of 25mg/ml of glycerol and 3% glycerol. Suitably the liposomes comprise an effective concentration of the compound of the disclosure. Suitably the liposomes comprise around 10⁻⁴ M of a compound of the disclosure, or a combination thereof. Suitably therefore the disclosure provides a composition or formulation comprising a plurality of liposomes, said liposomes comprising a compound of the disclosure, or a combination thereof, soy lecithin (preferably 25mg/ml) and glycerol (preferably 3%).

Suitably the liposomes may be manufactured by the Mozafari Heating Method (Mozafari, M. R. "Nanoliposomes: preparation and analysis." Liposomes: Methods and Protocols, Volume 1: Pharmaceutical Nanocarriers (2010): 29-50. Suitably such a method comprises (a) admixing the compound of the disclosure with glycerol, suitably with a 50% glycerol solution, (b) admixing the mixture of step (a) with soy lecithin, suitably with 350mg of soy lecithin in an aqueous solution, (c) heating the mixture of step (b) whilst stirring to form liposomes, suitably at around 60 °C and at around 800RPM for around 40 minutes, (d) annealing the liposomes, suitably by placing in 40 °C water for around 1-2 hours, and (e) sonicating the liposomes, suitably for around 30 minutes in a sonicator bath.

Suitably the liposomes have an average diameter of around 150 to 250 nm, suitably around 175nm to 225nm, suitably around 190 nm to 210 nm, suitably around 200 nm. Suitably the size of the liposomes is determined by using Dynamic light scattering (DLS), for example using a Malvern Zetasizer Nano. Suitably the liposomes have a polydispersity index of between 0.25 to 0.35, suitably 0.26 to 0.33, suitably 0.26 to 0.31, suitably 0.26 to 0.30, suitably 0.26 to 0.29, suitably 0.26 to 0.28, suitably around 0.26.

In the compositions of the disclosure, the carrier with the one or more insecticidal compounds may for example be maintained as a wettable powder, wettable granule, emulsifiable concentrate, suspension concentrate, microemulsion, capsule suspension, dry microcapsule, tablet or gel or be suspended, dispersed, emulsified or otherwise brought in a suitable liquid medium (such as water or another suitable aqueous, organic or oily medium) so as to provide a (concentrated) liquid composition of the disclosure that has a stability that allows the composition of the disclosure to be suitably stored or (where necessary after further dilution) applied to the intended site of action. Suitably the composition of the disclosure can be transported and/or stored prior to final use, optionally (and usually preferably) as a suitable liquid concentrate, dry powder, tablet, capsule suspension, slurry or "wet cake", which can be suitably diluted, dispersed, suspended, emulsified or otherwise suitably reconstituted by the end user prior to final use. The composition of the disclosure allows to be applied to the intended site of action using any suitable or desired manual or mechanical technique such as spraying, pouring, dripping, brushing, coating, dripcoating, applying as small droplets, a mist or an aerosol or any other suitable technique. In one example, said intended site of action is an intact living insect, even more preferably an insect surface.

The composition may be a bait composition for ingestion by the target insect. A bait composition may comprise one or more phagostimulants, i.e. a substance which will entice the insect to ingest the compound. Phagostimulants may include artificial sweeteners, amino acids, other peptides or proteins and carbohydrates (e.g. glucose, fructose, sucrose, maltose) etc. Examples include honey, syrups and aqueous solutions of sucrose.

Commercially available base formulations may also be suitable for use in formulating the compounds described in this specification, such as Armid^{®} FMPC (Akzo Nobel).

The composition may comprise one or more synergists, i.e. compounds which increase the efficacy of insecticides against their targets, often by inhibiting an insect's ability to metabolise the active agent. Common synergists include piperonyl butoxide and MGK-264 (n-octyl bicycloheptane dicarboximide), or peptidase inhibitors.

The composition may comprise one or more agents which promote stability of the insecticidal compounds of the disclosure. Suitably the one or more agents which promote stability may prevent degradation of the insecticidal compounds of the disclosure. Suitable agents which prevent degradation of the insecticidal compounds of the disclosure may inhibit or reduce the activity of enzymes, suitably of enzymes which act to degrade proteins, suitably proteases, for example. Suitably therefore the composition may comprise one or more protease inhibitors, suitably which may be selected from: Bowman-Birk Inhibitors, Kunitz Inhibitors, Cystatin, Trypsin Inhibitors, Serpins, Tannins, Proteinase Inhibitor-II (PI-II), and Alpha-Al1.

The composition may further comprise one or more additional, attractants, sterilizing agents, acaricides, nematicides, fungicides, growth-regulating substances or herbicides.

In some examples, the compositions may comprise one or more further agricultural chemicals or agrochemicals such as herbicides (e.g. contact herbicides or systemic herbicides, including herbicides for household use), fungicides (e.g. contact fungicides or systemic fungicides, including fungicides for household use), nematicides (e.g. contact nematicides or systemic nematicides, including nematicides for household use) and other pesticides or biocides (for example agents for killing insects or snails); as well as fertilizers; growth regulators such as plant hormones; micro-nutrients, safeners, pheromones; repellants; insect baits; and/or active principles that are used to modulate (i.e. increase, decrease, inhibit, enhance and/or trigger) gene expression (and/or other biological or biochemical processes) in or by the targeted plant (e.g. the plant to be protected or the plant to be controlled), such as nucleic acids (e.g., single stranded or double stranded RNA, as for example used in the context of RNAi technology) and other factors, proteins, chemicals, etc. known per se for this purpose, etc.

Examples of such agrochemicals will be clear to the skilled person; and for example include, without limitation: glyphosate, paraquat, metolachlor, acetochlor, mesotrione, 2,4-D,atrazine, glufosinate, sulfosate, fenoxaprop, pendimethalin, picloram, trifluralin, bromoxynil, clodinafop, fluroxypyr, nicosulfuron, bensulfuron, imazetapyr, dicamba, imidacloprid, thiamethoxam, fipronil, chlorpyrifos, deltamethrin, lambda-cyhalotrin, endosulfan, methamidophos, carbofuran, clothianidin, cypermethrin, abamectin, diflufenican, spinosad, indoxacarb, bifenthrin, tefluthrin, azoxystrobin, thiamethoxam, tebuconazole, mancozeb, cyazofamid, fluazinam, pyraclostrobin, epoxiconazole, chlorothalonil, copper fungicides, trifloxystrobin, prothioconazole, difenoconazole, carbendazim, propiconazole, thiophanate, sulphur, boscalid, chlorantraniliprole and other known agrochemicals or any suitable combination(s) thereof.

### Methods of Producing the Insecticidal Compounds

The insecticidal compounds described herein may be produced by any method known in the art for the production of peptides. In one example, the insecticidal compounds may be produced by a chemical method, suitably a chemical synthesis method.

Any suitable chemical method may be used to synthesis the insecticidal compounds of the disclosure.

Suitably the insecticidal compounds are peptides. Suitably therefore they are synthesised by solid-state peptide synthesis. Suitably such synthesis may be carried out by using commercially available machines such as the Biotage Initiator+ Alstra microwave-assisted peptide synthesiser or the CEM Liberty Prime microwave-assisted peptide synthesiser.

In one example of the present disclosure, the insecticidal compound is a peptide of the formula: [Pyr]-LTFTSSWGG-[NH₂] (SEQ ID NO:2). Suitably this peptide may be synthesised by the method defined in the examples. Suitably by the method defined in figure 2.

Further described herein is an isolated insecticidal compound produced by a process of the disclosure. Suitably an isolated insecticidal compound of formula (I) produced by a process of the disclosure.

Further described herein is a method of producing a composition as disclosed herein, at least comprising the steps of: (a) obtaining at least one insecticidal compound of formula (l), and (b) formulating the insecticidal compound into a composition.

Further described, but not covered by the wording of the claims, herein is a method of producing and/or manufacturing a variant of the insecticidal compound of formula (I). Such may comprise the steps of: (i) modifying the peptide Z of formula (I) by adding, replacing or deleting at least one amino acid; (ii) assessing the so created variant for its insecticidal activity and - optionally - at least one property selected from the group consisting of biostability, chemical stability, bioavailability, solubility (including the ability to form stable formulations), producibility, and production costs. Where the insecticidal activity is reduced in comparison to the unmodified peptide Z, repeating the process of steps (i) and (ii) until a variant with improved insecticidal activity is obtained. Where the insecticidal activity is improved in comparison to the unmodified peptide Z, the method may include further steps of manufacturing, isolating and purifying the variant. Screening for insecticidal activity and the other above described properties can be conducted as described below or generally known to the person skilled in the art.

In one example is provided a method for manufacturing a variant of the insecticidal compound of the disclosure, comprising the steps of:
a) modifying the peptide Z by adding, replacing or deleting at least one amino acid;
b) assessing the ability of the variant for its insecticidal activity and - optionally - at least one property selected from the group consisting of biostability, chemical stability, bioavailability, solubility (including the ability to form stable formulations), producibility, and production costs;
c) where the insecticidal activity is reduced in comparison to the insecticidal compound comprising the unmodified peptide Z, repeating the process of steps (a) and (b) until a variant with improved insecticidal activity is obtained;
d) where the insecticidal activity is improved in comparison to the insecticidal compound comprising the unmodified peptide Z, further manufacturing, isolating and purifying the variant.

Suitably the step of obtaining at least one insecticidal compound comprises (a) chemically synthesising the insecticidal compound.

Suitable compositions are described hereinabove. Suitable manufacturing methods for formulating the composition are known in the art and include, but are not limited to, high or low shear mixing, wet or dry milling, drip-casting, encapsulating, emulsifying, coating, encrusting, pilling, extrusion granulation, fluid bed granulation, co-extrusion, spray drying, spray chilling, atomization, addition or condensation polymerization, interfacial polymerization, in situ polymerization, coacervation, spray encapsulation, cooling melted dispersions, solvent evaporation, phase separation, solvent extraction, sol-gel polymerization, fluid bed coating, pan coating, melting, passive or active absorption or adsorption.

The features disclosed in the foregoing description, or in the accompanying drawings, expressed in their specific forms or in terms of a means for performing the disclosed function, or a method or process for obtaining the disclosed results, as appropriate, may, separately, or in any combination of such features, be utilised for realising the disclosed subject matter in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

### Brief Description of the Figures

The present disclosure will now be discussed with reference to the accompanying figures in which:

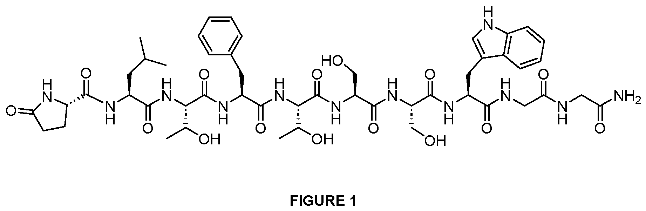

**Figure 1** shows the chemical structure of SB-P-86;
**Figure 2** shows the chemical synthesis route for synthesising SB-P-86;
**Figure 3** shows the lepidoptera artificial diet assay experimental set up;
**Figure 4** shows activity data of unformulated SB-P-86 against *Spodoptera frugiperda* after 168 hours. One larva was assayed per cell. (n=10);
**Figure 5** shows activity data of unformulated SB-P-86 against *Plutella xylostella* at 120 hours using leaf dip assay conducted by FERA.

### EXAMPLES

The disclosure will now be demonstrated and further described by way of the following non-limiting examples.

### General Procedures

All amino acids are of L-configuration unless otherwise stated. Standard Fmoc-protected amino acids were purchased from CEM Corporation or Pepceuticals. Suppliers of specialist amino acids are indicated as and when appropriate, as is peptide synthesis resin supplier. Peptide-grade DMF was purchased from Rathburn.

Peptides were synthesised on a Biotage Initiator+ Alstra microwave-assisted peptide synthesiser or a CEM Liberty Prime microwave-assisted peptide synthesiser as specified.

High-resolution mass spectrometry (HRMS) was performed on a Bruker microTOF-Q II (ESI+).

Peptides were purified on a reverse-phase Dionex HPLC system equipped with Dionex P680 pumps and a Dionex UVD170U UV-vis detector (monitoring at 214 nm and 280 nm), using a Phenomenex, Gemini, C18, 5 µm, 250 x 21.2 mm column. Gradients were performed using solvents consisting of A (H₂O + 0.1 % TFA) and B (MeCN + 0.1 % TFA) and fractions were lyophilised on a Christ Alpha 2-4 LO plus freeze dryer.

Pure peptides were analysed on a Shimadzu reverse-phase HPLC (RP-HPLC) system equipped with Shimadzu LC-20AT pumps, a SIL-20A autosampler and a SPD-20A UV-vis detector (monitoring at 214 nm and 280 nm) using a Phenomenex, Aeris, 5 µm, peptide XB-C18, 150 x 4.6 mm column at a flow rate of 1 mL/min. RP-HPLC gradients were run using a solvent system consisting of solution A (100% H₂O + 0.1% TFA) and B (100% MeCN + 0.1% TFA). Typically, two gradients were used to characterise each peptide; a gradient from 5% to 95% solution B over 20 min (incorporating a 2 min hold at 5% solution B and a 5 min wash at 95% solution B at the start and end of the gradient respectively) and a gradient from 5-95% solution B over 50 min (incorporating a 5 min hold at 5% solution B and a 5 min wash at 95% solution B at the start and end of the gradient respectively). In some cases, specialised gradients were used and this is indicated where appropriate. Analytical RP-HPLC data is reported as column retention time (tR) in minutes (min). Analytical columns were maintained at ambient temperature.

LC-MS analysis was performed on a Thermo Scientific LCQ Fleet quadropole mass spectrometer of m/z range 50-2000 Da with an ESI source coupled to a Dionex Ultimate 3000 LC. Analyses were performed on a ReprosilGold 120 C18, 3µm 150 x 4 mm column using a linear gradient of buffer A (95/5 H₂O/MeCN with 0.1% v/v TFA) to buffer B (95/5 MeCN/H₂O with 0.1% v/v TFA) over 20 min (incorporating a 2 min hold at 0% solution B and a 5 min wash at 100% solution B at the start and end of the gradient respectively). In cases where a specialised analytical RP-HPLC gradient was used to characterise a compound, an analogous LC-MS gradient was used. Analytical RP-HPLC and LC-MS samples were injected as 25 µL of a stock with concentration of 1mg/mL in H₂O/MeCN with 0.1% v/v TFA. LC-MS column oven temperature was maintained at 30 °C.

High-resolution mass spectrometry (HRMS) of pure peptides was performed on a Bruker microTOF-Q II (ESI+).

Proton nuclear magnetic resonance spectra (¹H NMR) for the purpose of peptide content calculations were recorded on an AVANCE III 400 Bruker (400 MHz). Proton chemical shifts are expressed in parts per million (ppm, δ scale) and are referenced to residual protium in the NMR solvent (CDCl₃, δ 7.26; CD₃OD, δ 3.31 and D₂O, δ 4.79). The following abbreviations were used to describe peak patterns when appropriate: br = broad, s = singlet, d = doublet, t = triplet, q = quadruplet, m = multiplet. Coupling constants, J, are reported in Hertz unit (Hz).

### General procedure for automated peptide synthesis

### Biotage Initiator + Alstra Synthesiser:

Fmoc-protected amino acids were prepared as a 0.2 M (0.1 mmol syntheses), 0.5 M (0.2mmol syntheses) or 0.7 M (0.5 mmol syntheses) solution in DMF. 5 equivalents of amino acid (relative to the resin loading) were used during coupling cycles. Oxyma and diisopropyl carbodiimide (DIC) were prepared as 0.2 M (0.1 mmol syntheses), 0.5 M (0.2 mmol syntheses) or 0.7 M (0.5 mmol syntheses) solutions in DMF. 5 equivalents of Oxyma and 5 equivalents of DIC (relative to resin loading) were used during coupling cycles. For Fmoc-deprotections, a solution of 20% morpholine (with 5% formic acid) in DMF was used. Coupling reactions were performed under microwave heating at 90 °C for 2 min with the exception of Fmoc-Cys(Trt)-OH, Fmoc-His(Trt)-OH and Fmoc-Arg(PBf)-OH. Coupling of Fmoc-Cys(Trt) and Fmoc-His(Trt)-OH was performed for 10 min at 50 °C. Coupling of Fmoc-Arg(Pbf)-OH was performed for 2 successive cycles (double-coupled) for 2 min at 90 °C. Microwave-assisted Fmoc deprotections were carried out at 90 °C for 1 min.

### CEM Liberty Blue Synthesiser

Fmoc-protected amino acids were prepared as a 0.2 M solution in NBP (Tamisolve). 5 equivalents of amino acid (relative to the resin loading) were used during coupling cycles. Oxyma was prepared as a 0.5 M solution in NBP. DIC was prepared as 5 M solution in NBP. 5 equivalents of Oxyma and 5 equivalents of DIC (relative to resin loading) were used during coupling cycles. For Fmoc-deprotections, a solution of 20% pyrrolidine was used. Coupling reactions and Fmoc-deprotections were performed under microwave heating at 90 °C for 2 min and 1 min respectively with the exception of Fmoc-Cys(Trt)-OH, Fmoc-His(Trt)-OH and Fmoc-Arg(Pbf)-OH. Coupling of Fmoc-Cys(Trt) and Fmoc-His(Trt)-OH was performed for 5 min at 50 °C. Coupling of Fmoc-Arg(Pbf)-OH was performed for 2 successive cycles of 5 min at 75 °C.

### General Procedure for TFA Cleavage of Peptides

Typically, cleavage tests of peptides were performed by taking ~3 mg of dried resin beads and treating them with TFA/TIS/water (95:2.5:2.5) for 3 h. The filtrate was drained, concentrated and then triturated in cold diethyl ether (Et₂O). The triturate was dissolved in acetonitrile/water, then analysed by RP-HPLC/LC-MS.

Peptides were typically cleaved from the resin in bulk by gently rocking the resin at rt in a cleavage cocktail of TFA/TIS/H₂O (95:2.5:2.5) for 3 h before being drained and the TFA blown off with a steady stream of N₂ gas. Peptides containing cysteine or tryptophan residues were cleaved from the resin using a cleavage cocktail of TFA/TIS/H₂O/DODT (94:2.5:1:2.5) for 3 h. In all cases the crude peptide was triturated with cold Et₂O. Et₂O was removed from the resulting crude peptide pellet under a steady stream of nitrogen. The crude peptide was then redissolved in H₂O/MeCN and purified by RP-HPLC.

A specific synthesis route for SB-P-86 is shown in Figure 2.

Unless otherwise stated herein, peptides were synthesised according to the general procedures above, in line with the exemplary procedures below, or were ordered directly. SB-P-86 peptide synthesis was outsourced and ordered directly from a commercial supplier.

### MORTALITY ASSESSMENT METHODS

### Lepidoptera artificial diet assay

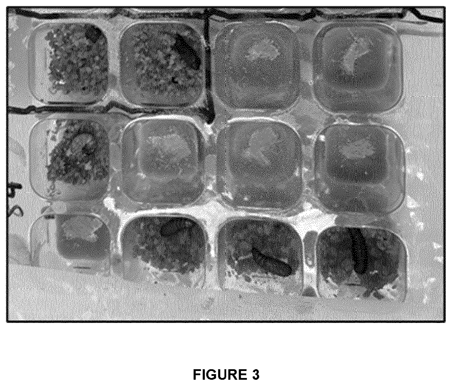

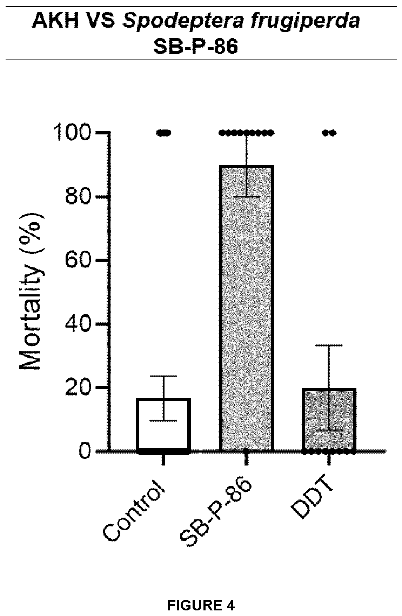

Frontier Lepidoptera Artificial diet was made up by preparing agar at 1% and then mixing it with artificial diet powder. Peptide solution was aliquoted in 10 cells and diet poured straight onto it. The final concentration of the peptide was 1x10⁻⁴ M, and each peptide was aliquoted in 10 cells. Once the diet solidified and cooled down, one L1 *Spodoptera frugiperda* larvae was infested in each cell. The trays were sealed with heat and let larva were allowed to develop for 168 hrs. Finally, pupation and mortality were scored for. The lepidoptera artificial diet assay set up is shown in Figure 3. Lepidoptera artificial diet assays were performed with SB-P-86, and compared to a negative control vehicle and the insecticide dichlorodiphenyltrichloroethane (DDT). The activity of SB-P-86 is shown in Figure 4; SB-P-86 has much higher activity whilst being eco-friendly and targeted to specific pests unlike broad spectrum chemical insecticides such as DDT.

### Leaf dip assay

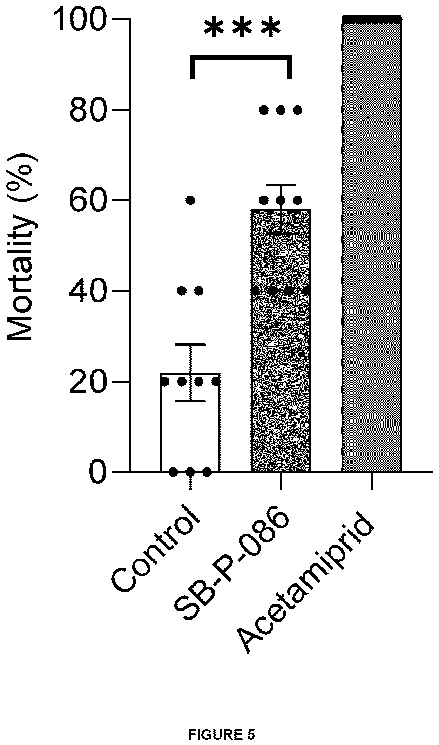

Peptides were tested in a leaf dip bioassay using Chinese cabbage on IRAC method 018 against *Plutella xylostella.* Peptide concentration at 2 × 10⁻⁴ M in a solution of 0.5% DMSO, 8% ACN, 0.1% Tween-80 in water. Acetamiprid (pesticide) was tested at 0.005%. Vehicle control comprised 0.5% DMSO, 8% ACN, 0.1% Tween-80 in water. Peptide SB-P-86 was tested by a Contract Research Organisation, FERA Science Ltd. The activity of SB-P-86 is shown in Figure 5; SB-P-86 has good activity whilst being eco-friendly and targeted to specific pests unlike broad spectrum chemical insecticides such as Acetamiprid.

### References

A number of publications are cited in order to more fully describe and disclose the disclosure and the state of the art to which the disclosure pertains. Full citations for these references are provided below.
∘ Koyama T, Terhzaz S, Naseem MT, Nagy S, Rewitz K, Dow JAT, Davies SA, Halberg KV. A nutrient-responsive hormonal circuit mediates an inter-tissue program regulating metabolic homeostasis in adult Drosophila. Nat Commun. 2021
∘ Kean L, Cazenave W, Costes L, Broderick KE, Graham S, Pollock VP, Davies SA, Veenstra JA, Dow JA. Two nitridergic peptides are encoded by the gene capability in Drosophila melanogaster. Am J Physiol Regul Integr Comp Physiol. 2002
∘ Yeoh JGC, Pandit AA, Zandawala M, Nässel DR, Davies SA, Dow JAT. DINeR: Database for Insect Neuropeptide Research. Insect Biochem Mol Biol. 2017
∘ Ahn SJ, Corcoran JA, Vander Meer RK, Choi MY. Identification and Characterization of GPCRs for Pyrokinin and CAPA Peptides in the Brown Marmorated Stink Bug, Halyomorpha halys (Hemiptera: Pentatomidae). Front Physiol. 2020
∘ Audsley N and Down RE, G protein coupled receptors as targets for next generation pesticides. Insect Biochem Molec 67: 27-37 (2015).
∘ Halberg KA, Terhzaz S, Cabrero P, Davies SA and Dow JAT, Tracing the evolutionary origins of insect renal function. Nat Commun 6 (2015).
∘ Dow JA, Insights into the Malpighian tubule from functional genomics. J Exp Biol 212: 435-445 (2009).
∘ Huesmann GR, Cheung CC, Loi PK, Lee TD, Swiderek KM and Tublitz NJ, Amino acid sequence of CAP2b, an insect cardioacceleratory peptide from the tobacco hawkmoth Manduca sexta. FEBS Lett 371: 311-314 (1995).
∘ Davies SA, Cabrero P, Povsic M, Johnston NR, Terhzaz S and Dow JAT, Signaling by drosophila capa neuropeptides. Gen Comp Endocr 188: 60-66 (2013).
∘ Terhzaz S, Teets NM, Cabrero P, Henderson L, Ritchie MG, Nachman RJ, Dow JAT, Denlinger DL and Davies SA, Insect capa neuropeptides impact desiccation and cold tolerance. Proc Natl Acad Sci 201501518 (2015).
∘ Terhzaz S, Alford L, Yeoh JGC, Marley R, Dornan AT, Dow JAT and Davies SA, Renal neuroendocrine control of desiccation and cold tolerance by Drosophila suzukii. Pest Manag Sci 74: 800-810 (2017).
∘ Predel R, Wegener C, Biology of the CAPA peptides in insects. Cell Mol Life Sci 63: 2477-2490 (2006).
∘ Lamango NS, Nachman RJ, Hayes TK, Strey A and Isaac RE, Hydrolysis of insect neuropeptides by an angiotensin converting enzyme from the housefly, M. domestica. Peptides 18: 47-52 (1997).
∘ Terhzaz S, Cabrero P, Robben JH, Radford JC, Hudson BD, Milligan G, Dow JA and Davies SA, Mechanism and function of Drosophila capa GPCR: a desiccation stress-responsive receptor with functional homology to human neuromedinU receptor. PLoS One 7(1): e29897 (2012).
∘ Blackman RL and Eastop VF, Aphids on the World's Crops: An Identification Guide, John Wiley & Sons Ltd, Chichester, UK. (2000).
∘ Dow JAT, Maddrell SHP, Gortz A, Skaer NJV, Brogan S and Kaiser K, The Malpighian tubules of Drosophila melanogaster - a novel phenotype for studies of fluid secretion and its control. J Exp Biol 197: 421-428 (1994).
∘ Davies SA, Huesmann GR, Maddrell SH, O'Donnell MJ, Skaer NJ, Dow JAT and Tublitz NJ, CAP2b, a cardioacceleratory peptide, is present in Drosophila and stimulates tubule fluid secretion via cGMP. Am J Physiol 269: R1321-R1326 (1995).
∘ Beyenbach KW, Skaer H and Dow JA, The developmental, molecular, and transport biology of Malpighian tubules. Annu Rev Entomol 55: 351-374 (2010).
∘ Sadeghi A., Van Damme, E.J.M. and Smagghe G. (2009) Evaluation of the susceptibility of the pea aphid, Acyrthosiphon pisum, to a selection of novel biorational insecticides using an artificial diet. Journal of Insect Science 9:65.
∘ Van Emden F (2009). Artificial diet for aphids - thirty years' experience. REDIA, XCII: 163-167

## Claims

1. Use of a compound having the formula (I) below, or a salt or solvate thereof:
R¹-Y¹-Z-Y²-R² (1)
wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 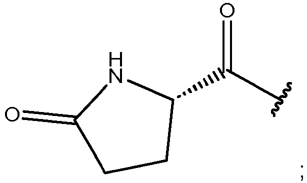 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) or a composition comprising a compound of formula (I) in admixture with one or more solvents, carriers, diluents, adjuvants, preservatives, dispersants, emulsifying agents, or synergists, as an insect control agent, or as a plant protection agent for protecting a plant or part thereof against insects.

2. The use of a compound or a composition according to claim 1 against:
a) dipteran insects, preferably wherein the dipteran insects are selected from: *D. melanogaster or D. suzukii;*
b) hemipteran insects, such as aphids, preferably wherein the hemipteran insects are selected from: *Aphis fabae, Aphis gossypii, Acyrthosiphon pisum, Myzus persicae or Rhopalosiphum padi;* and/or
c) lepidopteran insects, preferably wherein the lepidopteran insects are selected from: *Plutella xylostella* or *Spodoptera frugiperda;*
d) blattodean insects; and/or
e) coleopteran insects,
preferably wherein the use of a compound according to claim 8 is against *Plutella xylostella* or *Spodoptera frugiperda* insects.

3. The use of a compound or a composition according to claim 1 or 2, wherein R¹ is selected from hydrogen, acyl or fatty acyl, or phosphate or sulphate, or R¹ is a pyroglutamate group of the formula: 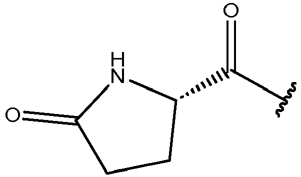 wherein acyl or fatty acyl are optionally substituted by a sugar moiety, phosphate or sulphate.

4. The use of a compound or a composition according to claim 1, 2 or 3, wherein R¹ is selected from hydrogen, formyl, acetyl (Ac), propanoyl, butanoyl, palmitoyl, butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, valeryl, and a pyroglutamate group of the formula: 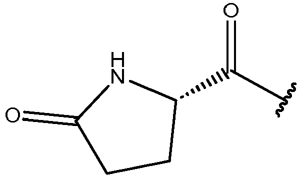
preferably wherein R¹ is selected from hydrogen, or palmitoyl, or pyroglutamate, more
preferably wherein R¹ is pyroglutamate.

5. The use of a compound or a composition according to any one of the preceding claims, wherein:
Y¹ is absent; and/or
wherein Y² is present and is selected from: GR, RG, KR, RK, K, R, and G; and/or
wherein R² is NH₂.

6. The use of a compound or a composition according to claim 1, wherein the compound has the formula:
[Pyr]-LTFTSSWGG-[NH₂] (SEQ ID NO:2).

7. A method of increasing insect mortality, the method comprising contacting an insect or insect population with a compound having the formula (I) below, or a salt or solvate thereof:
R¹-Y¹-Z-Y²-R² (I)
wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 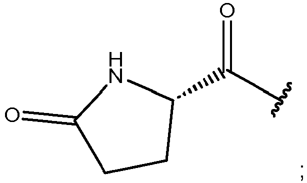 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

8. A method of inhibiting or preventing infestation of a plant by insects, the method comprising contacting the plant or a part thereof, or a site on which the plant is growing or is intended to be grown, with a compound having the formula (I) below, or a salt or solvate thereof:
R¹-Y¹-Z-Y²-R² (I)
wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 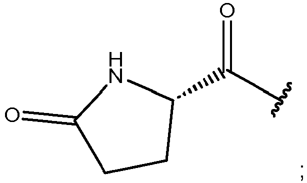 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

9. The method according to claim 8, wherein the compound or composition is contacted with the plant or part thereof, or a site on which the plant is growing or is intended to be grown, while the plant or part is free or substantially free of insects.

10. A method of reducing or treating an insect infestation of a plant, or of reducing insect load on a plant, the method comprising contacting the plant or a part thereof, or a site on which the plant is growing, with a compound having the formula (I) below, or a salt or solvate thereof:
R¹-Y¹-Z-Y²-R² (I)
wherein:
R¹ is hydrogen (which may be designated "H-" or "Hy-"), C₁₋₄alkyl (e.g. methyl, ethyl, propyl, butyl), formyl, acyl, fatty acyl, a sugar moiety, phosphate or sulphate, or R¹ is a pyroglutamate group of the formula: 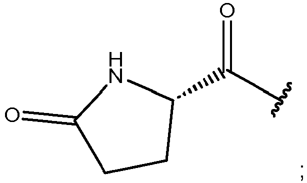 and any alkyl, formyl, acyl or fatty acyl may optionally be substituted with one or more groups selected from oxo or C₁₋₆alkyl, or a sugar moiety, phosphate or sulphate;
Y¹ is absent or is a peptide comprising from 1 to 2 amino acids;
Z is a peptide according to formula:
LTFTSSWGG (SEQ ID NO:1);
Y² is absent or is a peptide comprising from 1 to 2 amino acids;
R² is NH₂, NR^{2a}H, NR^{2a}R^{2b}, OH or OR^{2a}; wherein each of R^{2a} and R^{2b} if present are independently C₁₋₆-alkyl (e.g. methyl, ethyl, propyl, butyl, pentyl or hexyl) , or a composition comprising a compound of formula (I) in admixture with one or more solvents, carriers, diluents, adjuvants, preservatives, dispersants, emulsifying agents, or synergists.

11. The method according to any of claims 7-10, wherein R¹ is selected from hydrogen, acyl or fatty acyl, or phosphate or sulphate, or R¹ is a pyroglutamate group of the formula: 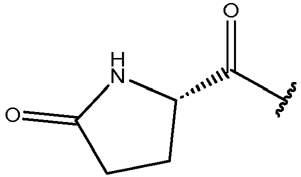 wherein acyl or fatty acyl are optionally substituted by a sugar moiety, phosphate or sulphate.

12. The method according to any of claims 7-11 , wherein R¹ is selected from hydrogen, formyl, acetyl (Ac), propanoyl, butanoyl, palmitoyl, butyryl, cerotoyl, decanoyl, docosenoyl, dodecanoyl, eleostearoyl, heptanoyl, hexanoyl, icosanoyl, icosenoyl, lignoceroyl, linoleoyl, lipoyl, myristoleoyl, nonanoyl, octadecanoyl, ocatanoyl, palmitoleoyl, stearoyl, undecanoyl, valeryl, and a pyroglutamate group of the formula: 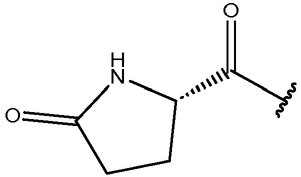
preferably wherein R¹ is selected from hydrogen, or palmitoyl, or pyroglutamate, more
preferably wherein R¹ is pyroglutamate.

13. The method according to any of claims 7-12, wherein:
Y¹ is absent; and/or
wherein Y² is present and is selected from: GR, RG, KR, RK, K, R, and G; and/or
wherein R² is NH₂.

14. The method according to any of claims 7, 8 or 10, having the formula:
[Pyr]-LTFTSSWGG-[NH₂] (SEQ ID NO:2).

15. The method according to claims 8 or 9 , wherein the site on which the plant is growing or intended to be grown may comprise an agricultural site suitable for growing plants, preferably the site comprises a field.

16. The method according to any of claims 7-15, wherein the insects are:
a) dipteran insects, preferably wherein the dipteran insects are selected from: *D. melanogaster or D. suzukii;*
b) hemipteran insects, such as aphids, preferably wherein the hemipteran insects are selected from *Aphis fabae, Aphis gossypii, Acyrthsiphon pisum, Myzus persicae* or *Rhopalosiphum padi;*
c) lepidopteran insects, preferably wherein the lepidopteran insects are selected from: *Plutella xylostella* or *Spodoptera frugiperda;*
d) blattodean insects; and/or
e) coleopteran insects,
preferably wherein the insects are *Plutella xylostella* or *Spodoptera frugiperda* insects.

17. The method according to any of claims 7-16, wherein contacting the insect or insect population, plant or part thereof, or site, comprises watering feeding, spraying, atomizing, foaming, fogging, culturing in hydroculture, culturing in hydroponics, coating, submerging, injecting and/or encrusting the insect or insect population, plant or part thereof, or site with the compound or composition.

18. The method according to any of claims 7-17, wherein the insect or insect population, plant or part thereof, or site, is contacted with an effective concentration of the compound, preferably a concentration of between 10⁻³ to 10⁻⁹ M.

19. The method according to any of claims 1 to 18, wherein the composition is an aqueous composition.

20. The method according to any of claims 1 to 19, wherein the composition comprises an effective amount of the compound.

## Patentansprüche

1. Verwendung einer Verbindung mit der nachstehenden Formel (I) oder eines Salzes oder Solvats davon:
R¹-Y¹-Z-Y²-R² (I)
wobei:
R¹ Wasserstoff (der als "H-" oder "Hy-" bezeichnet sein kann), C₁₋₄-Alkyl (z. B. Methyl, Ethyl, Propyl, Butyl), Formyl, Acyl, Fettacyl, eine Zuckereinheit, Phosphat oder Sulfat ist oder R¹ eine Pyroglutamatgruppe der Formel: 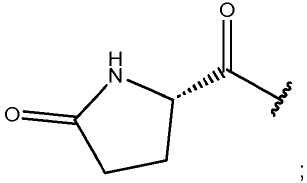 ist und ein beliebiges Alkyl, Formyl, Acyl oder Fettacyl optional mit einer oder mehreren Gruppen substituiert sein kann, die ausgewählt sind aus Oxo oder C₁₋₆-Alkyl oder einer Zuckereinheit, Phosphat oder Sulfat;
Y¹ fehlt oder ein Peptid umfassend von 1 bis 2 Aminosäuren ist;
Z ein Peptid gemäß der folgenden Formel ist:
LTFTSSWGG (SEQ ID NO:1);
Y² fehlt oder ein Peptid umfassend von 1 bis 2 Aminosäuren ist;
R² NH₂, NR^{2a}H, NR^{2a}R^{2b}, OH oder OR^{2a} ist; wobei jedes von R^{2a} und R^{2b}, falls vorhanden, unabhängig C₁₋₆-Alkyl (z. B. Methyl, Ethyl, Propyl, Butyl, Pentyl oder Hexyl) oder eine Zusammensetzung umfassend eine Verbindung der Formel (I) im Gemisch mit einem oder mehreren Lösungsmitteln, Trägern, Verdünnungsmitteln, Adjuvanzien, Konservierungsmitteln, Dispergiermitteln, Emulgatoren oder Synergisten, als Insektenbekämpfungsmittel, oder als Pflanzenschutzmittel zum Schützen einer Pflanze oder eines Teils davon vor Insekten sind.

2. Verwendung einer Verbindung oder einer Zusammensetzung nach Anspruch 1 gegen:
a) Diptera-Insekten, bevorzugt, wobei die Diptera-Insekten ausgewählt sind aus: *D. melanogaster oder D. suzukii;*
b) Hemiptera-Insekten, wie z. B. Blattläuse, vorzugsweise, wobei die Hemiptera-Insekten ausgewählt sind aus: *Aphis fabae, Aphis gossypii, Acyrthosiphon pisum, Myzus persicae oder Rhopalosiphum padi,* und/oder
c) Lepidoptera-Insekten, bevorzugt, wobei die Lepidoptera-Insekten ausgewählt sind aus: *Plutella xylostella* oder *Spodoptera frugiperda;*
d) Blattodea-Insekten; und/oder
e) Coleoptera-Insekten,
bevorzugt, wobei die Verwendung einer Verbindung nach Anspruch 8 gegen *Plutella xylostella-* oder *Spodoptera frugiperda*-Insekten erfolgt.

3. Verwendung einer Verbindung oder einer Zusammensetzung nach Anspruch 1 oder 2, wobei R¹ ausgewählt ist aus Wasserstoff, Acyl oder Fettacyl oder Phosphat oder Sulfat oder R¹ eine Pyroglutamatgruppe der Formel: 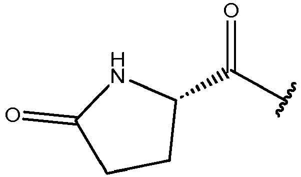 ist, wobei Acyl oder Fettacyl optional durch eine Zuckereinheit, Phosphat oder Sulfat substituiert sind.

4. Verwendung einer Verbindung oder einer Zusammensetzung nach Anspruch 1, 2 oder 3, wobei R¹ ausgewählt ist aus Wasserstoff, Formyl, Acetyl (Ac), Propanoyl, Butanoyl, Palmitoyl, Butyryl, Cerotoyl, Decanoyl, Docosenoyl, Dodecanoyl, Eleostearoyl, Heptanoyl, Hexanoyl, Icosanoyl, Icosenoyl, Lignoceroyl, Linoleoyl, Lipoyl, Myristoleoyl, Nonanoyl, Octadecanoyl, Ocatanoyl, Palmitoleoyl, Stearoyl, Undecanoyl, Valeryl und einer Pyroglutamatgruppe der Formel: 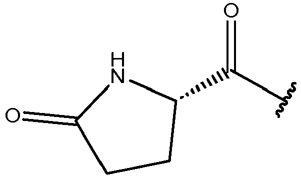 bevorzugt, wobei R¹ ausgewählt ist aus Wasserstoff oder Palmitoyl oder Pyroglutamat, bevorzugter, wobei R¹ Pyroglutamat ist.

5. Verwendung einer Verbindung oder einer Zusammensetzung nach einem der vorhergehenden Ansprüche, wobei:
Y¹ fehlt; und/oder
wobei Y² vorhanden ist und ausgewählt ist aus: GR, RG, KR, RK, K, R und G; und/oder
wobei R² NH₂ ist.

6. Verwendung einer Verbindung oder einer Zusammensetzung nach Anspruch 1, wobei die Verbindung die folgende Formel aufweist:
[Pyr]-LTFTSSWGG-[NH₂] (SEQ ID NO:2).

7. Verfahren zum Erhöhen der Insektenmortalität, wobei das Verfahren das Inkontaktbringen eines Insekts oder einer Insektenpopulation mit einer Verbindung mit der nachstehenden Formel (I) oder einem Salz oder Solvat davon umfasst:
R¹-Y¹-Z-Y²-R² (I)
wobei:
R¹ Wasserstoff (der als "H-" oder "Hy-" bezeichnet sein kann), C₁₋₄-Alkyl (z. B. Methyl, Ethyl, Propyl, Butyl), Formyl, Acyl, Fettacyl, eine Zuckereinheit, Phosphat oder Sulfat ist oder R¹ eine Pyroglutamatgruppe der Formel: 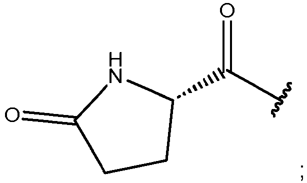 ist und ein beliebiges Alkyl, Formyl, Acyl oder Fettacyl optional mit einer oder mehreren Gruppen substituiert sein kann, die ausgewählt sind aus Oxo oder C₁₋₆-Alkyl oder einer Zuckereinheit, Phosphat oder Sulfat;
Y¹ fehlt oder ein Peptid umfassend von 1 bis 2 Aminosäuren ist;
Z ein Peptid gemäß der folgenden Formel ist:
LTFTSSWGG (SEQ ID NO:1);
Y² fehlt oder ein Peptid umfassend von 1 bis 2 Aminosäuren ist;
R² NH₂, NR^{2a}H, NR^{2a}R^{2b}, OH oder OR^{2a} ist; wobei jedes von R^{2a} und R^{2b}, falls vorhanden, unabhängig C₁₋₆-Alkyl (z. B. Methyl, Ethyl, Propyl, Butyl, Pentyl oder Hexyl) oder eine Zusammensetzung umfassend eine Verbindung der Formel (I) im Gemisch mit einem oder mehreren Lösungsmitteln, Trägern, Verdünnungsmitteln, Adjuvanzien, Konservierungsmitteln, Dispergiermitteln, Emulgatoren oder Synergisten sind.

8. Verfahren zum Hemmen oder Verhindern des Befalls einer Pflanze durch Insekten, wobei das Verfahren das Inkontaktbringen der Pflanze oder eines Teils davon oder eines Ortes, an dem die Pflanze wächst oder wachsen soll, mit einer Verbindung mit der nachstehenden Formel (I) oder einem Salz oder Solvat davon:
R¹-Y¹-Z-Y²-R² (I)
umfasst, wobei:
R¹ Wasserstoff (der als "H-" oder "Hy-" bezeichnet sein kann), C₁₋₄-Alkyl (z. B. Methyl, Ethyl, Propyl, Butyl), Formyl, Acyl, Fettacyl, eine Zuckereinheit, Phosphat oder Sulfat ist oder R¹ eine Pyroglutamatgruppe der Formel: 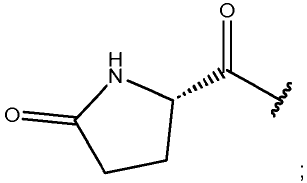 ist und ein beliebiges Alkyl, Formyl, Acyl oder Fettacyl optional mit einer oder mehreren Gruppen substituiert sein kann, die ausgewählt sind aus Oxo oder C₁₋₆-Alkyl oder einer Zuckereinheit, Phosphat oder Sulfat;
Y¹ fehlt oder ein Peptid umfassend von 1 bis 2 Aminosäuren ist;
Z ein Peptid gemäß der folgenden Formel ist:
LTFTSSWGG (SEQ ID NO:1);
Y² fehlt oder ein Peptid umfassend von 1 bis 2 Aminosäuren ist;
R² NH₂, NR^{2a}H, NR^{2a}R^{2b}, OH oder OR^{2a} ist; wobei jedes von R^{2a} und R^{2b}, falls vorhanden, unabhängig C₁₋₆-Alkyl (z. B. Methyl, Ethyl, Propyl, Butyl, Pentyl oder Hexyl) oder eine Zusammensetzung umfassend eine Verbindung der Formel (I) im Gemisch mit einem oder mehreren Lösungsmitteln, Trägern, Verdünnungsmitteln, Adjuvanzien, Konservierungsmitteln, Dispergiermitteln, Emulgatoren oder Synergisten sind.

9. Verfahren nach Anspruch 8, wobei die Verbindung oder Zusammensetzung mit der Pflanze oder einem Teil davon oder einem Ort, an dem die Pflanze wächst oder wachsen soll, in Kontakt gebracht wird, während die Pflanze oder der Teil frei oder im Wesentlichen frei von Insekten ist.

10. Verfahren zum Reduzieren oder Behandeln des Insektenbefalls einer Pflanze oder zum Reduzieren der Insektenbelastung einer Pflanze, wobei das Verfahren das Inkontaktbringen der Pflanze oder eines Teils davon oder eines Ortes, an dem die Pflanze wächst, mit einer Verbindung mit der nachstehenden Formel (I) oder einem Salz oder Solvat davon:
R¹-Y¹-Z-Y²-R² (I)
umfasst, wobei:
R¹ Wasserstoff (der als "H-" oder "Hy-" bezeichnet sein kann), C₁₋₄-Alkyl (z. B. Methyl, Ethyl, Propyl, Butyl), Formyl, Acyl, Fettacyl, eine Zuckereinheit, Phosphat oder Sulfat ist oder R¹ eine Pyroglutamatgruppe der Formel: 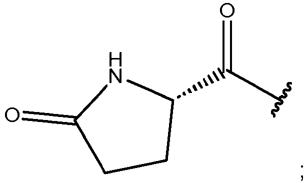 ist und ein beliebiges Alkyl, Formyl, Acyl oder Fettacyl optional mit einer oder mehreren Gruppen substituiert sein kann, die ausgewählt sind aus Oxo oder C₁₋₆-Alkyl oder einer Zuckereinheit, Phosphat oder Sulfat;
Y¹ fehlt oder ein Peptid umfassend von 1 bis 2 Aminosäuren ist;
Z ein Peptid gemäß der folgenden Formel ist:
LTFTSSWGG (SEQ ID NO:1);
Y² fehlt oder ein Peptid umfassend von 1 bis 2 Aminosäuren ist;
R² NH₂, NR^{2a}H, NR^{2a}R^{2b}, OH oder OR^{2a} ist; wobei jedes von R^{2a} und R^{2b}, falls vorhanden, unabhängig C₁₋₆-Alkyl (z. B. Methyl, Ethyl, Propyl, Butyl, Pentyl oder Hexyl) oder eine Zusammensetzung umfassend eine Verbindung der Formel (I) im Gemisch mit einem oder mehreren Lösungsmitteln, Trägern, Verdünnungsmitteln, Adjuvanzien, Konservierungsmitteln, Dispergiermitteln, Emulgatoren oder Synergisten sind.

11. Verfahren nach einem der Ansprüche 7-10, wobei R¹ ausgewählt ist aus Wasserstoff, Acyl oder Fettacyl oder Phosphat oder Sulfat oder R¹ eine Pyroglutamatgruppe der Formel: 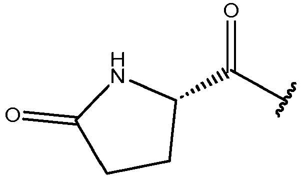 ist, wobei Acyl oder Fettacyl optional durch eine Zuckereinheit, Phosphat oder Sulfat substituiert sind.

12. Verfahren nach einem der Ansprüche 7-11, wobei R¹ ausgewählt ist aus Wasserstoff, Formyl, Acetyl (Ac), Propanoyl, Butanoyl, Palmitoyl, Butyryl, Cerotoyl, Decanoyl, Docosenoyl, Dodecanoyl, Eleostearoyl, Heptanoyl, Hexanoyl, Icosanoyl, Icosenoyl, Lignoceroyl, Linoleoyl, Lipoyl, Myristoleoyl, Nonanoyl, Octadecanoyl, Ocatanoyl, Palmitoleoyl, Stearoyl, Undecanoyl, Valeryl und einer Pyroglutamatgruppe der Formel: 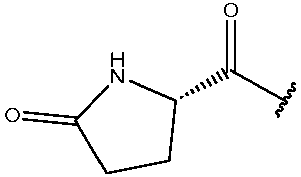 bevorzugt, wobei R¹ ausgewählt ist aus Wasserstoff oder Palmitoyl oder Pyroglutamat, bevorzugter, wobei R¹ Pyroglutamat ist.

13. Verfahren nach einem der Ansprüche 7-12, wobei:
Y¹ fehlt; und/oderwobei Y² vorhanden ist und ausgewählt ist aus: GR, RG, KR, RK, K, R und G;
und/oderwobei R² NH₂ ist.

14. Verfahren nach einem der Ansprüche 7, 8 oder 10, mit der Formel:
[Pyr]-LTFT-SSVVGG-[NH₂] (SEQ ID NO:2).

15. Verfahren nach Anspruch 8 oder 9, wobei der Ort, an dem die Pflanze wächst oder wachsen soll, einen landwirtschaftlichen Ort umfassen kann, der für den Anbau von Pflanzen geeignet ist, bevorzugt, der Ort einen Acker umfasst.

16. Verfahren nach einem der Ansprüche 7-15, wobei die Insekten folgende sind:
a) Diptera-Insekten, bevorzugt, wobei die Diptera-Insekten ausgewählt sind aus: *D. melanogaster oder D. suzukii;*
b) Hemiptera-Insekten, wie z. B. Blattläuse, bevorzugt, wobei die Hemiptera-Insekten ausgewählt sind aus *Aphis fabae, Aphis gossypii, Acyrthsiphon pisum, Myzus persicae* oder *Rhopalosiphum padi;*
c) Lepidoptera-Insekten, bevorzugt, wobei die Lepidoptera-Insekten ausgewählt sind aus: *Plutella xylostella* oder *Spodoptera frugiperda;*
d) Blattodea-Insekten; und/oder
e) Coleoptera-Insekten,
bevorzugt, wobei die Insekten *Plutella xylostella-* oder *Spodoptera frugiperda*-Insekten sind.

17. Verfahren nach einem der Ansprüche 7-16, wobei das Inkontaktbringen des Insekts oder der Insektenpopulation, der Pflanze oder eines Teils davon oder des Ortes das Bewässern, Nähren, Sprühen, Zerstäuben, Schäumen, Vernebeln, Kultivieren in Hydrokultur, Kultivieren in Hydroponik, Beschichten, Eintauchen, Injizieren und/oder Verkrusten des Insekts oder der Insektenpopulation, der Pflanze oder eines Teils davon oder des Ortes mit der Verbindung oder Zusammensetzung umfasst.

18. Verfahren nach einem der Ansprüche 7-17, wobei das Insekt oder die Insektenpopulation, die Pflanze oder ein Teil davon oder der Ort mit einer wirksamen Konzentration der Verbindung, bevorzugt einer Konzentration von zwischen 10⁻³ bis 10⁻⁹ M, in Kontakt gebracht wird.

19. Verfahren nach einem der Ansprüche 1 bis 18, wobei die Zusammensetzung eine wässrige Zusammensetzung ist.

20. Verfahren nach einem der Ansprüche 1 bis 19, wobei die Zusammensetzung eine wirksame Menge der Verbindung umfasst.

## Revendications

1. Utilisation d'un composé présentant la formule (I) ci-dessous, ou d'un sel ou d'un solvate de celui-ci :
R¹-Y¹-Z-Y²-R² (I)
où :
R¹ est un hydrogène (qui peut être désigné par « H- » ou « Hy- »), un alkyle en C₁₋₄ (par exemple un méthyle, un éthyle, un propyle, un butyle), un formyle, un acyle, un acyle gras, une fraction de sucre, un phosphate ou un sulfate, ou R¹ est un groupe pyroglutamate de formule : 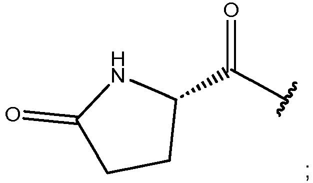 et tout alkyle, formyle, acyle ou acyle gras peut éventuellement être substitué par un ou plusieurs groupes choisis parmi un oxo ou un alkyle en C₁₋₆, ou une fraction de sucre, un phosphate ou un sulfate ;
Y¹ est absent ou est un peptide comprenant 1 à 2 acides aminés ;
Z est un peptide selon la formule :
LTFTSSWGG (SEQ ID NO:1);
Y² est absent ou est un peptide comprenant 1 à 2 acides aminés ;
R² est NH₂, NR^{2a}H, NR^{2a}R^{2b}, OH ou OR^{2a} ; chacun de R^{2a} et R^{2b}, si présent, étant indépendamment un alkyle en C₁₋₆ (par exemple un méthyle, un éthyle, un propyle, un butyle, un pentyle ou un hexyle) ou une composition comprenant un composé de formule (I) en mélange avec un ou plusieurs solvants, supports, diluants, adjuvants, conservateurs, dispersants, agents émulsifiants ou synergistes, en tant qu'agent de lutte contre les insectes ou en tant qu'agent phytosanitaire pour protéger une plante ou une partie de celle-ci contre les insectes.

2. Utilisation d'un composé ou d'une composition selon la revendication 1 contre :
a) des insectes diptères, de préférence lesdits insectes diptères étant choisis parmi : *D. melanogaster ou D. suzukii ;*
b) des insectes hémiptères, tels que des pucerons, de préférence lesdits insectes hémiptères étant choisis parmi : *Aphis fabae, Aphis gossypii, Acyrthosiphon pisum, Myzus persicae ou Rhopalosiphum padi ;* et/ou
c) des insectes lépidoptères, de préférence lesdits insectes lépidoptères étant choisis parmi : *Plutella xylostella* ou *Spodoptera frugiperda ;*
d) des insectes blattoptères ; et/ou
e) des insectes coléoptères,
de préférence ladite utilisation d'un composé selon la revendication 8 étant contre les insectes *Plutella xylostella* ou *Spodoptera frugiperda.*

3. Utilisation d'un composé ou d'une composition selon l'une des revendications 1 ou 2, dans laquelle R¹ est choisi parmi un hydrogène, un acyle ou un acyle gras, ou un phosphate ou un sulfate, ou R¹ est un groupe pyroglutamate de formule : 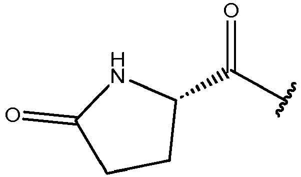 dans laquelle l'acyle ou l'acyle gras est éventuellement substitué par une fraction de sucre, un phosphate ou un sulfate.

4. Utilisation d'un composé ou d'une composition selon l'une des revendications 1, 2 ou 3, dans laquelle R¹ est choisi parmi un hydrogène, un formyle, un acétyle (Ac), un propanoyle, un butanoyle, un palmitoyle, un butyryle, un cérotoyle, un décanoyle, un docosénoyle, un dodécanoyle, un éléostéaroyle, un heptanoyle, un hexanoyle, un icosanoyle, un icosénoyle, un lignocéroyle, un linoléoyle, un lipoyle, un myristoléoyle, un nonanoyle, un octadécanoyle, un ocatanoyle, un palmitoléoyle, un stéaroyle, un undécanoyle, un valéryle et un groupe pyroglutamate de formule : 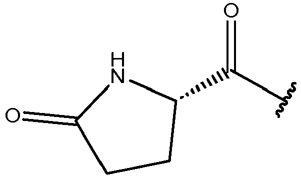 dans laquelle de préférence, R¹ est choisi parmi un hydrogène, ou un palmitoyle, ou un pyroglutamate, dans laquelle idéalement R¹ est un pyroglutamate.

5. Utilisation d'un composé ou d'une composition selon l'une quelconque des revendications précédentes, dans laquelle :
Y¹ est absent ; et/ou
dans laquelle Y² est présent et est choisi parmi : GR, RG, KR, RK, K, R et G ; et/ou
dans laquelle R² est NH₂.

6. Utilisation d'un composé ou d'une composition selon la revendication 1, dans laquelle le composé présente la formule :
[Pyr]-LTFTSSWGG-[NH₂] (SEQ ID NO:2).

7. Procédé d'augmentation de la mortalité des insectes, le procédé comprenant la mise en contact d'un insecte ou d'une population d'insectes avec un composé présentant la formule (I) ci-dessous, ou un sel ou un solvate de celui-ci :
R¹-Y¹-Z-Y²-R² (I)
où :
R¹ est un hydrogène (qui peut être désigné par « H- » ou « Hy- »), un alkyle en C₁₋₄ (par exemple un méthyle, un éthyle, un propyle, un butyle), un formyle, un acyle, un acyle gras, une fraction de sucre, un phosphate ou un sulfate, ou R¹ est un groupe pyroglutamate de formule : 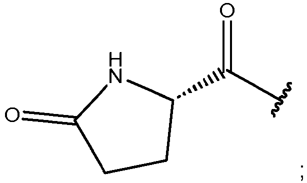 et tout alkyle, formyle, acyle ou acyle gras peut éventuellement être substitué par un ou plusieurs groupes choisis parmi un oxo ou un alkyle en C₁₋₆, ou une fraction de sucre, un phosphate ou un sulfate ;
Y¹ est absent ou est un peptide comprenant 1 à 2 acides aminés ;
Z est un peptide selon la formule :
LTFTSSWGG (SEQ ID NO:1);
Y² est absent ou est un peptide comprenant 1 à 2 acides aminés ;
R² est NH₂, NR^{2a}H, NR^{2a}R^{2b}, OH ou OR^{2a} ; chacun de R^{2a} et R^{2b}, si présent, étant indépendamment un alkyle en C₁₋₆ (par exemple un méthyle, un éthyle, un propyle, un butyle, un pentyle ou un hexyle), ou une composition comprenant un composé de formule (I) en mélange avec un ou plusieurs solvants, supports, diluants, adjuvants, conservateurs, dispersants, agents émulsifiants ou synergistes.

8. Procédé d'inhibition ou de prévention de l'infestation d'une plante par des insectes, le procédé comprenant la mise en contact de la plante ou d'une partie de celle-ci, ou d'un site sur lequel la plante est cultivée ou est destinée à être cultivée, avec un composé présentant la formule (I) ci-dessous, ou un sel ou un solvate de celui-ci :
R¹-Y¹-Z-Y²-R² (I)
où :
R¹ est un hydrogène (qui peut être désigné par « H- » ou « Hy- »), un alkyle en C₁₋₄ (par exemple un méthyle, un éthyle, un propyle, un butyle), un formyle, un acyle, un acyle gras, une fraction de sucre, un phosphate ou un sulfate, ou R¹ est un groupe pyroglutamate de formule : 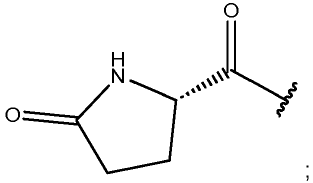 et tout alkyle, formyle, acyle ou acyle gras peut éventuellement être substitué par un ou plusieurs groupes choisis parmi un oxo ou un alkyle en C₁₋₆, ou une fraction de sucre, un phosphate ou un sulfate ;
Y¹ est absent ou est un peptide comprenant 1 à 2 acides aminés ;
Z est un peptide selon la formule :
LTFTSSWGG (SEQ ID NO:1);
Y² est absent ou est un peptide comprenant 1 à 2 acides aminés ;
R² est NH₂, NR^{2a}H, NR^{2a}R^{2b}, OH ou OR^{2a} ; chacun de R^{2a} ou R^{2b}, si présent, étant indépendamment un alkyle en C₁₋₆ (par exemple un méthyle, un éthyle, un propyle, un butyle, un pentyle ou un hexyle), ou une composition comprenant un composé de formule (I) en mélange avec un ou plusieurs solvants, supports, diluants, adjuvants, conservateurs, dispersants, agents émulsifiants ou synergistes.

9. Procédé selon la revendication 8, dans lequel le composé ou la composition est mis(e) en contact avec la plante ou une partie de celle-ci, ou un site sur lequel la plante est cultivée ou est destinée à être cultivée, tandis que la plante ou la partie est exempte ou sensiblement exempte d'insectes.

10. Procédé de réduction ou de traitement d'une infestation d'insectes sur une plante, ou de réduction de la charge d'insectes sur une plante, le procédé comprenant la mise en contact de la plante ou d'une partie de celle-ci, ou d'un site sur lequel la plante est cultivée, avec un composé présentant la formule (1) ci-dessous, ou un sel ou un solvate de celui-ci :
R¹-Y¹-Z-Y²-R² (I)
où :
R¹ est un hydrogène (qui peut être désigné par « H- » ou « Hy- »), un alkyle en C₁₋₄ (par exemple un méthyle, un éthyle, un propyle, un butyle), un formyle, un acyle, un acyle gras, une fraction de sucre, un phosphate ou un sulfate, ou R¹ est un groupe pyroglutamate de formule : 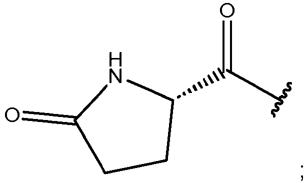 et tout alkyle, formyle, acyle ou acyle gras peut éventuellement être substitué par un ou plusieurs groupes choisis parmi un oxo ou un alkyle en C₁₋₆, ou une fraction de sucre, un phosphate ou un sulfate ;
Y¹ est absent ou est un peptide comprenant 1 à 2 acides aminés ;
Z est un peptide selon la formule :
LTFTSSWGG (SEQ ID NO:1):
Y² est absent ou est un peptide comprenant 1 à 2 acides aminés ;
R² est NH₂, NR^{2a}H, NR^{2a}R^{2b}, OH ou OR^{2a} ; chacun de R^{2a} et R^{2b}, si présent, étant indépendamment un alkyle en C₁₋₆ (par exemple un méthyle, un éthyle, un propyle, un butyle, un pentyle ou un hexyle), ou une composition comprenant un composé de formule (I) en mélange avec un ou plusieurs solvants, supports, diluants, adjuvants, conservateurs, dispersants, agents émulsifiants ou synergistes.

11. Procédé selon l'une quelconque des revendications 7 à 10, dans lequel R¹ est choisi parmi un hydrogène, un acyle ou un acyle gras, ou un phosphate ou un sulfate, ou R¹ est un groupe pyroglutamate de formule : 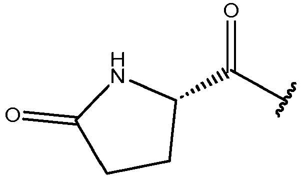 dans lequel l'acyle ou l'acyle gras est éventuellement substitué par une fraction de sucre, un phosphate ou un sulfate.

12. Procédé selon l'une quelconque des revendications 7 à 11, dans lequel R¹ est choisi parmi un hydrogène, un formyle, un acétyle (Ac), un propanoyle, un butanoyle, un palmitoyle, un butyryle, un cérotoyle, un décanoyle, un docosénoyle, un dodécanoyle, un éléostéaroyle, un heptanoyle, un hexanoyle, un icosanoyle, un icosénoyle, un lignocéroyle, un linoléoyle, un lipoyle, un myristoléoyle, un nonanoyle, un octadécanoyle, un ocatanoyle, un palmitoléoyle, un stéaroyle, un undécanoyle, un valéryle et un groupe pyroglutamate de formule : 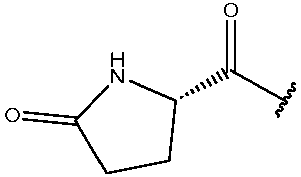 dans lequel de préférence, R¹ est choisi parmi un hydrogène, ou un palmitoyle, ou un pyroglutamate, dans lequel idéalement R¹ est un pyroglutamate.

13. Procédé selon l'une quelconque des revendications 7 à 12, dans lequel :
Y¹ est absent ; et/ou
dans lequel Y² est présent et est choisi parmi : GR, RG, KR, RK, K, R et G ; et/ou
dans lequel R² est NH₂.

14. Procédé selon l'une quelconque des revendications 7, 8 ou 10, présentant la formule :
[Pyr]-LTFTSSVWGG-[NH₂] (SEQ ID NO:2).

15. Procédé selon l'une des revendications 8 ou 9, dans lequel le site sur lequel la plante est cultivée ou est destinée à être cultivée peut comprendre un site agricole approprié pour cultiver des plantes, de préférence le site comprend un champ.

16. Procédé selon l'une quelconque des revendications 7 à 15, dans lequel les insectes sont :
a) des insectes diptères, de préférence lesdits insectes diptères étant choisis parmi : *D. melanogaster ou D. suzukii ;*
b) des insectes hémiptères, tels que des pucerons, de préférence lesdits insectes hémiptères étant choisis parmi *Aphis fabae, Aphis gossypii, Acyrthsiphon pisum, Myzus persicae* ou *Rhopalosiphum padi ;*
c) des insectes lépidoptères, de préférence lesdits insectes lépidoptères étant choisis parmi : *Plutella xylostella* ou *Spodoptera frugiperda ;*
d) des insectes blattoptères ; et/ou
e) des insectes coléoptères,
de préférence lesdits insectes étant des insectes *Plutella xylostella* ou *Spodoptera frugiperda.*

17. Procédé selon l'une quelconque des revendications 7 à 16, dans lequel la mise en contact de l'insecte ou de la population d'insectes, de la plante ou d'une partie de celle-ci, ou du site, comprend l'arrosage, l'alimentation, la pulvérisation, l'atomisation, la formation de mousse, la brumisation, la culture en hydroculture, la culture en hydroponie, l'enrobage, l'immersion, l'injection et/ou l'encroûtement de l'insecte ou de la population d'insectes, de la plante ou d'une partie de celle-ci, ou du site avec le composé ou la composition.

18. Procédé selon l'une quelconque des revendications 7 à 17, dans lequel l'insecte ou la population d'insectes, la plante ou une partie de celle-ci, ou le site, est mis(e) en contact avec une concentration efficace du composé, de préférence une concentration comprise entre 10⁻³ et 10⁻⁹ M.

19. Procédé selon l'une quelconque des revendications 1 à 18, dans lequel la composition est une composition aqueuse.

20. Procédé selon l'une quelconque des revendications 1 à 19, dans lequel la composition comprend une quantité efficace du composé.
